# FASCICULE DE BREVET EUROPEEN

(11) **EP 1 189 903 B1**
(45) Date de publication et mention de la délivrance du brevet: **28.05.2003**
(21) Numéro de dépôt: 00949571.4
(22) Date de dépôt: 29.06.2000
(51) Int. Cl.: C07D 471/04, C07D 215/38, A61K 31/4375, A61P 31/00

(54) **NOUVEAUX DERIVES DE LA BENZO[f]NAPHTYRIDINE, LEUR PREPARATION ET LES COMPOSITIONS LES CONTENANT**
BENZO[F]NAPHTHYRIDINDERIVATE, IHRE HERSTELLUNG UND SIE ENTHALTENDE PHARMAZEUTISCHE ZUSAMMENSTZUNGEN
NOVEL BENZO[F]NAPHTHYRIDINE, PREPARATION AND COMPOSITIONS CONTAINING THEM

(30) Priorité: 30.06.1999 FR 9908376; 12.08.1999 US 148212 P
(43) Date de publication de la demande: 27.03.2002
(73) Titulaire: Aventis Pharma S.A., 92160 Antony (FR)
(72) Inventeur: DESCONCLOIS, Jean-François, F-75012 Paris (FR); GENEVOIS-BORELLA, Arielle, F-94320 Thiais (FR); GIRARD, Philippe, F-91290 Arpajon (FR); KRYVENKO, Michel, F-75116 Paris (FR); LAVERGNE, Marc, Pierre, F-94520 Mandres les Roses (FR); MALLERON, Jean-Luc, F-91460 Marcoussis (FR); PICAUT, Guy, F-94800 Villejuif (FR); TABART, Michel, F-91290 la Norville (FR); WENTZLER, Sylvie, F-94550 Chevilly Larue (FR)
(86) Numéro de dépôt international: FR0001819
(87) Numéro de publication internationale: WO01002396

(56) Documents cités:
- EP-A- 0 431 991
- FR-A- 2 258 855
- US-A- 3 300 499

## Description

La présente invention concerne de nouveaux dérivés de la benzo[f]naphtyridine de formule générale : 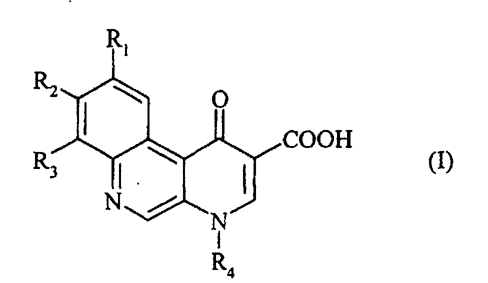 leurs sels, leur préparation et les compositions qui les contiennent.

Dans la demande de brevet EP 431 991 ont été décrits des dérivés de la benzo[b]naphtyridine de structure : 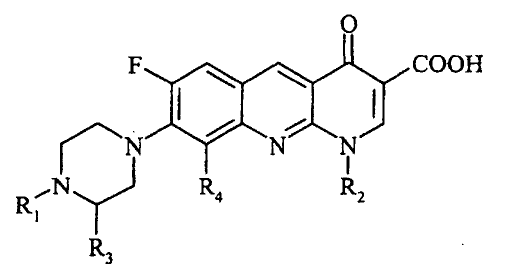 dans laquelle R₁ est H, hydroxy ou alkyle, R₂ est H, alkyle, fluoroalkyle, cycloalkyle, alkyloxy ou alkylamino, R₃ est phényle ou phénylalkyle éventuellement substitué, et R₄ est H ou un atome de fluor. Ces produits sont utiles comme agents antimicrobiens.

Dans la demande de brevet FR 2 258 855 ont été décrits des dérivés de la benzo[h]naphtyridine de structure : 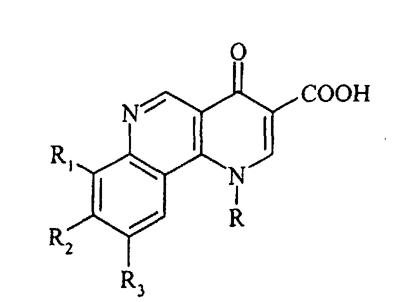 dans laquelle R est un radical alkyle, et R₁, R₂ et R₃, identiques ou différents, sont choisis parmi H, alkyle, polyhalogénoalkyle, halogène, alkoxy, nitro, alkylsulfone, sulfamide, ou bien R₁ et R₂ ou R₂ et R₃ peuvent être reliés entre eux pour constituer un nouveau cycle carboné saturé ou non, comportant 5 ou 6 atomes de carbone, la liaison entre ces radicaux eux-mêmes et le noyau de base pouvant avoir lieu par l'intermédiaire de un ou deux hétéroatome d'oxygène pour former un nouvel hétérocycle. Ces produits sont utiles comme agents antimicrobiens.

Le brevet américain US 3,300,499 décrit des dérivés de benzo[f]naphtyridine de structure : 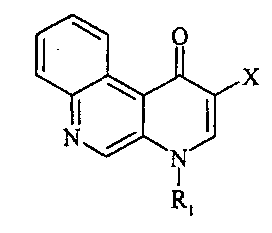 dans laquelle X est un groupe carboxy ou alkyloxycarbonyle, R₁ est un groupe alkyle ou alcényle, et les positions 5, 7, 8, 9 et 10 peuvent aussi porter des substituants choisis parmi alkyle, alkyloxy, hydroxy, halogène alkylamino et alkylthio.
Cependant, ces produits n'ont pas été trouvés actifs dans les tests d'activité bactériologique *in vitro* mis en oeuvre par la Demanderesse. Il a été trouvé maintenant que les dérivés de benzo[f]naphtyridine de formule générale (I) dans laquelle :
- R₁, R₂ et R₃, identiques ou différents, représentent un atome d'hydrogène, un atome d'halogène ou bien un groupe de formule générale (II) : 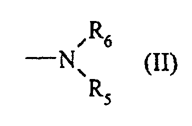
dans laquelle R₅ et R₆ forment ensemble avec l'atome d'azote auquel ils sont rattachés un hétérocycle à 5, 6 ou 7 chaînons dont 2 atomes de carbone peuvent éventuellement être liés entre eux par un pont contenant 1 ou 2 atomes de carbone, et contenant le cas échéant un autre hétéroatome choisi parmi l'azote, l'oxygène ou le soufre, ledit cycle pouvant en outre être substitué par un ou plusieurs radicaux phényle, phényle substitué (par un atome d'halogène, un radical alkyle, halogénoalkyle, alkyloxy, ou benzyloxy), benzyle, alkyle, hydroxy, aminoalkyle, alkylaminoalkyle, dialkylaminoalkyle, ou benzylaminoalkyle,
étant entendu que l'un des substituants R₁, R₂ ou R₃ est nécessairement un groupe de formule générale (II), et l'un au moins des deux autres représente un atome d'halogène, et
- R₄ représente un radical alkyle, fluoroalkyle, carboxyalkyle, cycloalkyle contenant 3 à 6 atomes de carbone, fluorophényle, difluorophényle, alkyloxy ou alkylamino, les radicaux alkyle cités ci-dessus étant droits ou ramifiés et contenant 1 à 4 atomes de carbone,
   ainsi que leurs sels, et le cas échéant leurs stéréoisomères, manifestent une activité antibactérienne particulièrement intéressante pour la voie topique.

Dans la formule générale ci-dessus les substituants halogène peuvent être choisis parmi le chlore, le fluor, le brome ou l'iode. Par ailleurs, lorsque R₅ et R₆ forment ensemble avec l'atome d'azote auquel ils sont attachés un hétérocycle à 5 à 7 chaînons, celui-ci peut être choisi à titre non limitatif parmi pyrrolidine, imidazolidine, pyrazolidine, pipéridine, pipérazine, morpholine, thiomorpholine, thiazolidine, perhydroazépine, perhydrodiazépine. En outre, les radicaux alkyle mentionnés ci-avant sont avantageusement choisis parmi méthyle, éthyle, propyle, isopropyle, *n*-butyle, *sec*-butyle ou encore *tert*-butyle.

Selon un aspect préféré de la présente invention, les dérivés de benzo[f]naphtyridine sont plus précisément choisis parmi les dérivés compris dans la formule générale suivante : 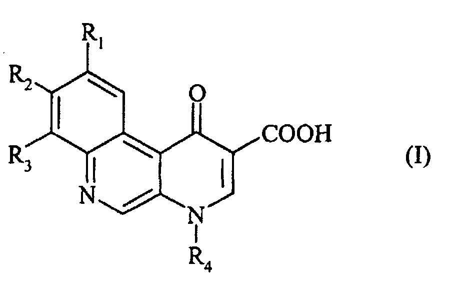 dans laquelle :
- R₁, R₂ et R₃, identiques ou différents, représentent un atome d'hydrogène, un atome d'halogène ou bien un groupe de formule générale (II): 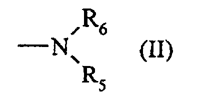 dans laquelle R₅ et R₆ forment ensemble avec l'atome d'azote auquel ils sont rattachés un hétérocycle à 6 ou 7 chaînons dont 2 atomes de carbone peuvent éventuellement être liés entre eux par un pont contenant 1 ou 2 atomes de carbone, et contenant le cas échéant un autre hétéroatome d'azote, ledit cycle pouvant en outre être substitué par un ou plusieurs radicaux phényle, phényle substitué (par un atome d'halogène, un radical alkyle, halogénoalkyle, alkyloxy, ou benzyloxy), ou alkyle,
   étant entendu que l'un des substituants R₁, R₂ ou R₃ est nécessairement un groupe de formule générale (II), et l'un au moins des deux autres représente un atome d'halogène, et
- R₄ représente un radical alkyle ou fluoroalkyle, les radicaux alkyle cités ci-dessus étant droits ou ramifiés et contenant 1 à 4 atomes de carbone,
ainsi que leurs sels, et le cas échéant leurs stéréôisomères.

Selon l'invention, les produits de formule générale (I) peuvent être obtenus par action d'une amine de formule générale : 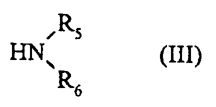 dans laquelle R₅ et R₆ sont définis comme précédemment,
sur un dérivé de benzo[f]naphtyridine de formule générale : 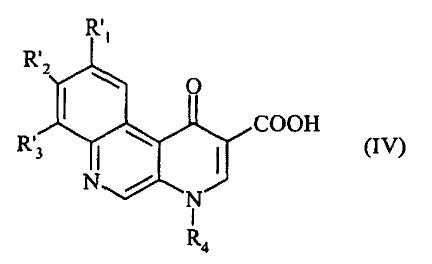 dans laquelle R₄ est défini comme précédemment ou représente un radical alkylamino protégé, et R'₁, R'₂ et R'₃ représentent indépendamment les uns des autres un atome d'hydrogène ou un atome d'halogène, étant entendu que deux au moins des substituants R'₁, R'₂ et R'₃ sont des atomes d'halogène,
les atomes d'halogène cités ci-dessus étant choisis parmi le fluor, le chlore, le brome ou l'iode,
puis séparation des produits aminés ainsi obtenus pour sélectionner la structure souhaitée.

L'action de l'amine de formule générale (III) s'effectue généralement en présence d'un excès de ce dérivé comme accepteur d'acide dans des solvants organiques convenables.

Il est possible d'opérer avec ou sans solvant, à une température comprise entre 20 et 150°C. Lorsque l'on opère en présence d'un solvant, la réaction s'effectue avantageusement dans des solvants polaires aprotiques, comme par exemple la pyridine, le diméthylformamide, le diméthylsulfoxyde ou l'acétonitrile. Il est également possible d'opérer en milieu aqueux.

Il peut être également avantageux d'opérer en présence d'un accepteur d'acide comme par exemple une base organique azotée (triéthylamine notamment), un carbonate alcalin (carbonate de sodium par exemple) ou un hydroxyde de métal alcalin ou alcalinoterreux.

Lorsque l'on fait agir l'amine de formule générale (III) sur le dérivé de benzo[f]naphtyridine de formule générale (IV), on obtient un mélange de produits monoaminés. Ces produits peuvent être séparés par des techniques classiques de séparation, par exemple par cristallisation ou encore par chromatographie préparative, en particulier Chromatographie Liquide Haute Performance (CLHP) ou chromatographie moyenne pression (« flash chromatography »), afin de sélectionner le produit de formule générale (I) souhaité.

Le dérivé de benzo[f]naphtyridine formule générale (IV) peut être obtenu à partir de l'ester correspondant de formule générale : 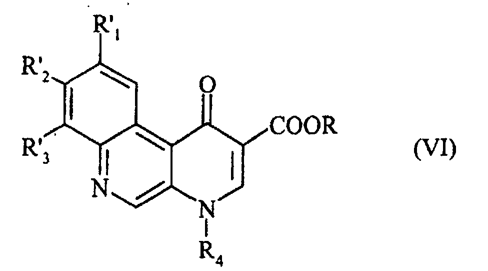 où R'₁, R'₂, R'₃ sont défini comme ci-avant, R₄ est défini comme précédemment ou représente un radical alkylamino protégé et R représente un radical alkyle contenant 1 à 4 atomes de carbone en chaîne droite ou ramifiée, par toute méthode connue pour obtenir un acide à partir d'un ester sans toucher au reste de la molécule.

La préparation de l'acide à partir de l'ester s'effectue généralement par saponification en présence de potasse ou de soude, en milieu aqueux ou hydroalcoolique, à une température comprise entre 20 et 100°C. Il est également possible d'opérer par hydrolyse acide à des températures telles que citées ci-avant.

Les composés de formules générales (IV) et (VI), qui peuvent être représentés sous la formule générale suivante : 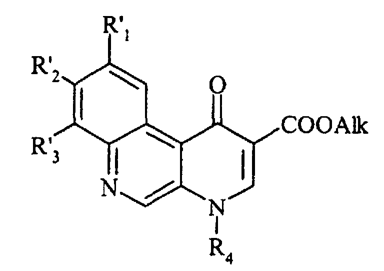 dans laquelle R'₁, R'₂, R'₃ et R₄ sont défini comme ci-avant, et Alk représente un atome d'hydrogène ou bien un radical alkyle contenant 1 à 4 atomes de carbone en chaîne droite ou ramifiée,
sont des intermédiaires de synthèse des benzo[f]naphtyridines de formule générale (I) selon la présente invention qui sont nouveaux, et constituent donc à ce titre un autre objet de la présente invention.

Selon l'invention, les dérivés de benzo[f]naphtyridine de formule générale (I) peuvent aussi être obtenus à partir de l'ester correspondant de formule générale : 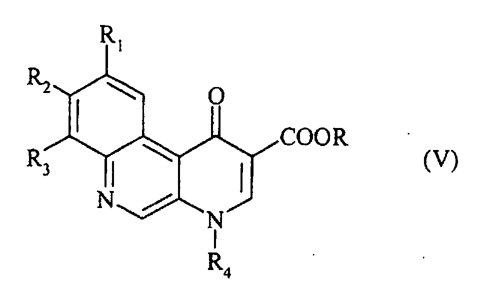 dans laquelle R, R₁, R₂ et R₃ sont définis comme précédemment, et R₄ est défini comme précédemment ou représente un radical alkylamino protégé, par toute méthode connue pour obtenir un acide à partir d'un ester sans toucher au reste de la molécule.

La préparation de l'acide à partir de l'ester s'effectue généralement par saponification en présence de potasse ou de soude, en milieu aqueux ou hydroalcoolique, à une température comprise entre 20 et 100°C. Il est également possible d'opérer par hydrolyse acide à des températures telles que citées ci-avant.

Lorsque R₄ représente un radical alkylamino protégé, le radical protecteur peut être tout groupement protecteur d'amino compatible avec la molécule. Il est particulièrement avantageux de choisir un radical protecteur qui peut être éliminé simultanément à l'hydrolyse de l'ester. La protection peut être réalisée par tout groupement compatible et dont la mise en oeuvre et l'élimination n'altère pas le reste de la molécule. Notamment, on opère selon les méthodes décrites par T.W. GREENE,

*Protective Groups in Organic Synthesis,* A. Wiley-Interscience Publication (1981), ou par Mc OMIE, *Protective Groups in Organic Chemistry,* Plenum Press (1973).

Le dérivé de la benzo[f]napthyridine de formule générale (V) peut être obtenu par action de l'amine de formule générale (III) sur l'ester correspondant de formule générale (VI) tels que définis ci-avant, selon la même méthode que celle décrite pour la réaction de l'amine de formule générale (III) avec le dérivé de benzo[f]naphtyridine de formule générale (IV). Il est entendu que dans l'alternative où l'on opère en milieu aqueux il est possible d'obtenir directement le produit de formule générale (I) sans isoler intermédiairement le dérivé de formule générale (V).

L'ester de benzo[f]naphtyridine de formule générale (VI) peut être obtenu de la façon suivante :
a) On fait agir un dérivé malonate de formule générale : 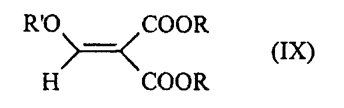 dans laquelle R est tel que défini précédemment et R', identique ou différent de R, représente un radical alkyle contenant 1 à 4 atomes de carbone en chaîne droite ou ramifiée, sur une aminoquinoléine de formule générale : 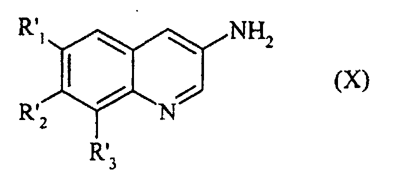 pour laquelle R'₁, R'₂ et R'₃ sont tels que défini ci-avant,
b) on cyclise le dérivé ainsi obtenu, de formule générale : 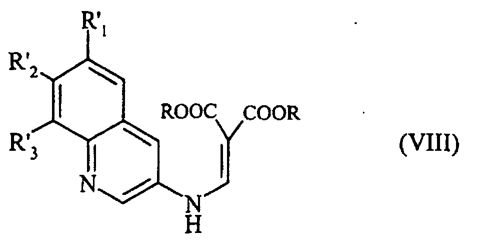 dans laquelle R'₁, R'₂, R'₃ et R sont définis comme précédemment, par cyclisation thermique de type Gould-Jacob,
c) puis on additionne un dérivé halogéné de formule R₄-Hal pour lequel R₄ est défini comme précédemment et Hal représente un atome d'halogène choisi parmi le chlore, le brome ou l'iode, sur le dérivé obtenu à l'étape précédente, de formule générale : 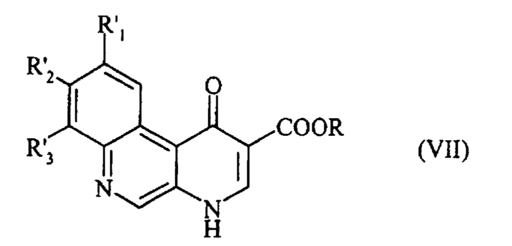
dans laquelle R'₁, R'₂, R'₃ et R sont définis comme précédemment.

Pour l'étape a) mentionnée précédemment, on opère généralement sans solvant à une température comprise entre 20 et 150°C, ou bien dans des solvants aromatiques, des solvants chlorés ou des éthers à une température comprise entre 20 et 110°C. A titre de solvant, on peut par exemple citer le xylène, le toluène, le chloroforme, le chlorure de méthylène ou encore le diphényléther.

La cyclisation thermique de type Gould-Jacob s'effectue généralement à température comprise entre 100 et 260°C dans le diphényl éther ou encore à l'eutectique du mélange phényl éther/biphényl (Angeles de la Cruz et coll., Tetrahedron, 48(29), pp. 6135-6150, 1992).

Enfin, la réaction selon l'étape c) telle que décrite précédemment s'effectue généralement en milieu basique à température comprise entre 20 et 150°C, dans un solvant organique approprié. A titre de base compatible, on peut utiliser des carbonates alcalins ou alcalino-terreux, par exemple le carbonate de potassium, le carbonate de sodium etc... On opère de préférence dans un solvant choisi parmi le diméthylformamide ou le diméthylsulfoxyde.

L'aminoquinoléine de formule générale (X) est un intermédiaire nouveau, utile dans la préparation des benzo[f]naphtyridines de formule générale (I) selon la présente invention, et constitue donc également un autre objet de la présente invention.

L'arninoquinoléine de formule générale (X) peut être obtenue de différente façons. Selon une première variante, l'aminoquinoléine de formule générale (X) peut être obtenue par réduction à partir de la nitroquinoléine correspondante de formule générale : 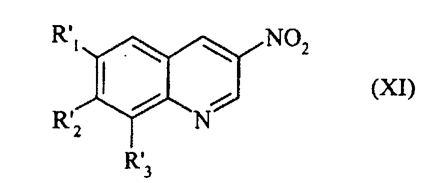 dans laquelle R'₁, R'₂, et R'₃ sont définis comme précédemment.

La réduction s'effectue selon les méthodes classiques connues ne touchant pas aux substituants halogénés, par exemple par hydrogénation catalytique en milieu acide en présence de nickel de Raney ou de palladium sur charbon, dans un alcool et à une température comprise entre 20 et 60°C. On peut utiliser à titre d'alcool du méthanol ou de l'éthanol. Une autre alternative consiste à opérer par action du chlorure stanneux en milieu aqueux acide à température comprise entre 20 et 100°C, ou encore par réduction par le fer en milieu aqueux acide et alcoolique à une température comprise entre 20 et 100°C. La solution aqueuse acide peut par exemple être une solution aqueuse d'acide chlorhydrique. La solution alcoolique peut par exemple être du méthanol ou de l'éthanol.

La nitroquinoléine de formule générale (XI) peut être obtenue par action d'acide méthazonique sur un dérivé benzaldéhyde de formule générale : 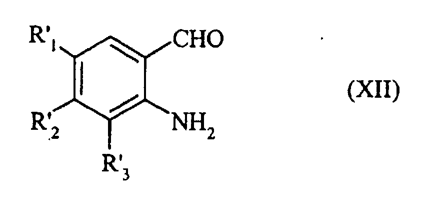 dans laquelle R'₁, R'₂, et R'₃ sont définis comme précédemment.

On opère généralement à une température comprise entre 20 et 50°C, en milieu aqueux acide qui peut être par exemple une solution aqueuse d'acide chlorhydrique.

Le dérivé de benzaldéhyde de formule générale (XII) peut être obtenue à partir de dérivé halogéné d'aniline correspondant de formule générale : 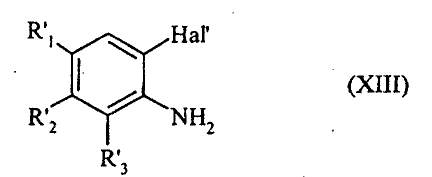 dans laquelle R'₁, R'₂, et R'₃ sont définis comme précédemment et Hal' représente un atome d'halogène choisi parmi le chlore, le brome et l'iode,
par action d'un organo-lithien dans un éther à une température comprise entre -75 et 20°C, suivie de l'addition de diméthylformamide. A titre non-limitatif, on peut citer le butylithium comme organo-lithien. De préférence, on opère dans le tétrahydrofurane.

Le produit de formule générale (XIII) est commercial ou bien il peut être obtenu par analogie avec les méthodes classiques d'halogénation des anilines, par exemple par action d'un halogénosuccinimide dans un solvant polaire aprotique à une température comprise entre -20 et 100°C. On peut par exemple opérer dans le diméthylformamide.

Selon une autre voie, l'aminoquinoléine de formule générale (X) peut être obtenue par dégradation de Hoffmann des amides à partir du dérivé carboxamide correspondant de formule générale : 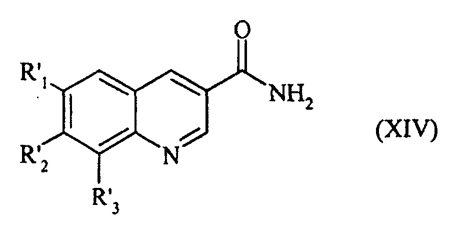 dans laquelle R'₁, R'₂, et R'₃ sont définis comme précédemment.

La réaction est généralement opérée par action d'un agent oxydant compatible avec la réaction de dégradation de Hoffmann des amides, à température comprise entre -20 et 100°C. A titre d'agent oxydant compatible avec la réaction, on peut par exemple utiliser l'hypobromite de sodium, le tétraacétate de plomb, des dérivés hypervalents de l'iode comme par exemple le 1,1-bis-(trifluoroacétoxy)iodobenzène etc...

On peut également opérer selon toute méthode connue pour obtenir une amine à partir d'un carboxamide sans toucher au reste de la molécule.

Le dérivé carboxamide de formule générale (XIV) peut être obtenu à partir du dérivé carboxylique correspondant de formule générale : 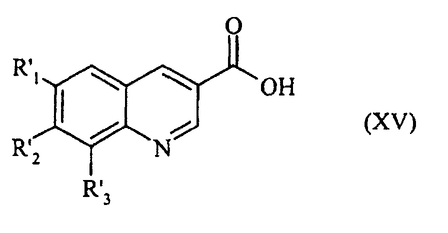 dans laquelle R'₁, R'₂, et R'₃ sont définis comme précédemment,
selon les méthodes classiques d'amidification connues pour obtenir un carboxamide à partir de l'acide correspondant sans toucher au reste de la molécule.

Notamment, on opère par addition de chlorure de thionyle dans un solvant chloré à une température comprise entre 20 et 80°C, puis on traite le chlorure d'acide ainsi obtenu par un courant d'ammoniac dans le chlorure de méthylène à température comprise entre -10 et 20°C. A titre de solvant chloré, on peut par exemple citer le chlororforme, le dichlorométhane etc...

Le dérivé de formule générale (XV) peut être obtenu à partir de l'ester correspondant de formule générale : 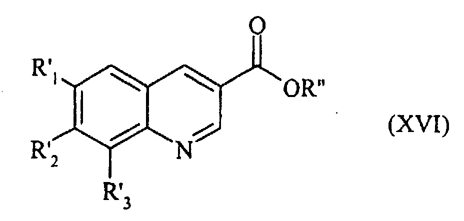 dans laquelle R'₁, R'₂, et R'₃ sont définis comme précédemment et R" représente un radical alkyle contenant 1 à 4 atomes de carbone en chaîne droite ou ramifiée,
par toute méthode connue pour obtenir un acide à partir d'un ester sans toucher au reste de la molécule.

La préparation de l'acide à partir de l'ester s'effectue généralement selon les méthodes décrites précédemment.

Le dérivé ester de formule générale (XVI) peut être obtenu par hydrogénation catalytique en milieu basique, en présence de palladium sur charbon, du dérivé halogéné de formule générale : 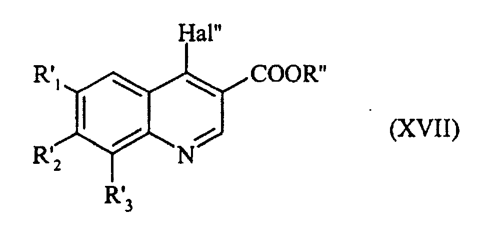 dans laquelle R", R'₁, R'₂, et R'₃ sont définis comme précédemment et Hal" représente un atome d'halogène choisi parmi le chlore, le brome ou l'iode.

On opère généralement dans un alcool, par exemple le méthanol ou l'éthanol, en présence d'une base telle que la triéthylamine ou la diisopropylamine, et de palladium sur charbon à une température comprise entre 20 et 60°C.

Le dérivé de formule générale (XVII) peut être obtenu par halogénation d'un dérivé de formule générale : 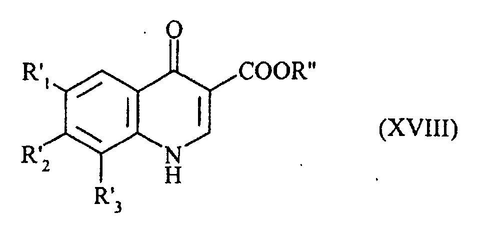 dans laquelle R", R'₁, R'₂, et R'₃ sont définis comme précédemment.

On opère généralement en présence d'oxychlorure de phosphore ou d'oxybromure de phosphore, à température comprise entre 20 et 120°C.

Le dérivé de formule générale (XVIII) peut être préparé à partir d'un dérivé de formule générale : 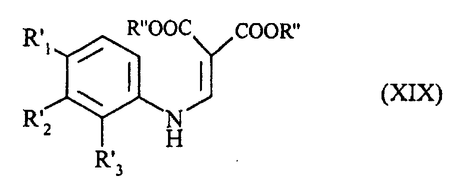 dans laquelle R", R'₁, R'₂, et R'₃ sont définis comme précédemment, selon la méthode décrite dans Angeles de la Cruz et coll., Tetrahedron, 48(29), pp. 6135-6150 (1992).
On opère également de façon analogue à la méthode décrite pour le passage du dérivé (VIII) au dérivé (VII) (étape b) telle que écrite précédemment), ou par toute autre méthode analogue connue de l'homme du métier.

Enfin, le dérivé de formule générale (XIX) peut être obtenu à partir du dérivé d'aniline de formule générale : 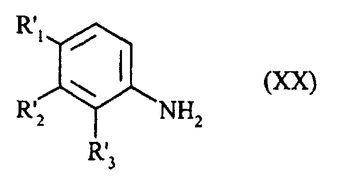 dans laquelle R'₁, R'₂, et R'₃ sont définis comme précédemment,
par action d'un dérivé malonate de formule générale : 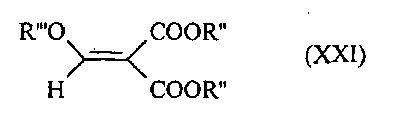 dans laquelle R" est tel que défini précédemment et R"', identique ou différent de R", représente un radical alkyle contenant 1 à 4 atomes de carbone en chaîne droite ou ramifiée, selon des méthodes analogues à celle décrite par Angeles de la Cruz et coll.,

Tetrahedron, 48(29), pp. 6135-6150 (1992).

On opère notamment dans des conditions opératoires analogues à celle décrites précédemment pour passer du dérivé de formule générale (X) au dérivé de formule générale (VIII).

Le dérivé aniline de formule générale (XX) est commercial ou bien il peut être obtenu selon les méthodes classiques, notamment par réduction du dérivé nitré correspondant en ne touchant pas aux substituants halogénés, ou bien le dérivé de formule générale (XX) peut être obtenu selon les méthodes classiques d'halogénation des anilines, comme cela a été précédemment décrit.

Selon l'invention, le cas échéant lorsque des formes stéréoisomères des dérivés de la benzo[f]naphtyridine de formule générale (I) existent et lorsque l'on veut obtenir ces stéréoisomères, la séparation des formes stéréoisomères des amines de formule générale (III) est effectuée, par toute méthode connue et compatible avec la molécule. A titre d'exemple, la séparation s'effectue par acylation au moyen d'un acide ou d'un dérivé réactif d'un acide chiral, séparation des isomères par chromatographie liquide hautes performances, puis désacylation selon la méthode décrite par P.G. Gasseman et coll., J. Am. Chem. Soc., 98(5), p.1275 (1976). Il est également possible d'effectuer la séparation des stéréoisomères par chromatographie liquide hautes performances sur phase chirale.

Les nouveaux produits selon la présente invention ainsi que leurs intermédiaires de synthèse peuvent être éventuellement purifiés par des méthodes physiques telles que la cristallisation ou la chromatographie.

Les produits selon la présente invention ainsi que leurs intermédiaires de formule générale (IV) peuvent être transformés en sels métalliques ou en sels d'addition avec les bases azotées selon les méthodes connues en soi. Ces sels peuvent être obtenus selon les méthodes habituelles qui n'altèrent pas le reste de la molécule, notamment par action d'une base métallique (par exemple alcaline ou alcalino-terreuse), d'ammoniac ou d'une amine sur un produit cité ci-dessus dans un solvant approprié tel qu'un alcool, un éther ou l'eau, ou par réaction d'échange avec un sel d'un acide organique. Le sel formé précipite après concentration éventuelle de sa solution, il est séparé par filtration, décantation et/ou lyophilisation.

Les nouveaux produits selon l'invention peuvent être également transformés en sels d'addition avec les acides. Les produits de formule générale (I) obtenus sous forme de ces sels, peuvent être libérés et transformés en sels d'autres acides selon les méthodes habituelles.

Comme exemples de sels pharmaceutiquement acceptables, peuvent être cités les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropyl-amine, N,N-diméthyléthanolamine, benzylamine, dicyclohexylamine, N-benzyl phénéthylamine, N,N'-dibenzyléthylènediamine, diphénylènediamine, benzhydrylamine, quinine, choline, arginine, lysine, leucine, dibenzylamine), ainsi que les sels d'addition avec des acides minéraux (chlorhydrates, bromhydrates, sulfates, nitrates, phosphates) ou organiques (succinates, fumarates, maléates, méthanesulfonates, p.toluènesulfonates, iséthionates).

Les nouveaux dérivés de benzo[f]naphtyridine de formule générale (I) selon la présente invention et leurs sels pharmaceutiquement acceptables présentent des propriétés antibactériennes particulièrement intéressantes. Ils manifestent une activité remarquable *in vitro* et *in* vivo sur les germes gram-positifs et plus particulièrement sur les germes résistants aux quinolones. Compte tenu de leur activité, ils sont tout particulièrement indiqués pour un usage par voie topique.

*In vitro,* les produits de formule générale (I) se sont montrés actifs à une concentration comprise entre 1 et 4 µg/cm3 sur *Staphylococcus aureus* IP 8203 et à une concentration comprise entre 1 et 8 µg/cm3 sur *Staphylococcus aureus* LF11C128B résistant aux quinolones.

*In vivo*, les produits se sont montrés actifs à une concentration comprise 2% et 5% dans une formulation de cétomacrogel et d'alcool benzylique, dans le modèle de contamination du cobaye à *Staphylococcus aureus* TCC 25923.

Enfin les produits selon l'invention ne présentent pas de toxicité aux doses utilisées. Le taux d'irritation cutanée mesuré chez le lapin, en formulation dans du cetomacrogol et de l'alcool benzylique est de 1 pour une formulation contenant 1% de produit dans l'excipient, comparé à 0,8 pour l'excipient seul. De plus une formulation contenant 10% de produit dans l'excipient ci-dessus n'a pas montré d'irritation supérieure chez le cobaye.

Les exemples suivants donnés à titre non limitatif illustrent la présente invention.

### Exemple 1

L'acide 8-chloro-7-fluoro-9-[4-(3-fluoro-4-méthylphényl)pipérazin-1-yl]-4-méthyl-1-oxo-1,4-dihydrobenzo[f][1,7]naphtyridine-2-carboxylique peut être obtenu selon la méthode suivante :
Une suspension agitée de 0,61 g de 8-chloro-7-fluoro-9-[4-(3-fluoro-4-méthylphényl)-pipérazin-1-yl]-4-méthyl-1-oxo-1,4-dihydro-benzo[f][1,7]naphtyridine-2-carboxylate d'éthyle dans 15 cm³ d'éthanol et 1,4 cm³ de potasse aqueuse 1N est chauffée au voisinage de 100°C pendant 11 heures. La solution obtenue est acidifiée à cette même température par 1,5 cm³ d'une solution aqueuse 1N d'acide acétique.
Après refroidissement, l'insoluble est essoré, puis lavé par 2 fois avec 5 cm³ d'eau, 1 fois 5 cm³ d'éthanol, et 2 fois 5 cm³ de diéthyl éther. Après une recristallisation dans 50 cm³ de diméthylformamide, on obtient 0,40 g d'acide 8-chloro-7-fluoro-9-[4-(3-fluoro-4-méthyl-phényl)-pipérazin-1-yl]-4-méthyl-1-oxo-1,4-dihydro-benzo[f][1,7]naphtyridine-2-carboxylique, sous forme d'un solide jaune fondant à 360°C.

Le 8-chloro-7-fluoro-9-[4-(3-fluoro-4-méthylphényl)pipérazin-1-yl]-4-méthyl-1-oxo-1,4-dihydrobenzo[f][1,7]naphtyridine-2-carboxylate d'éthyle est obtenu de la façon suivante :
Une suspension agitée de 1 g de 8-chloro-7,9-difluoro-4-méthyl-1-oxo-1,4-dihydro-benzo[f][1,7]naphtyridine-2-carboxylate d'éthyle et de 1,2 g de 1-(3-fluoro-4-méthyl-phényl)-pipérazine dans 15 cm³ de diméthylsulfoxyde est chauffée à une température voisine de 80°C. Au bout de 20 heures, on rajoute 0,6 g de 1-(3-fluoro-4-méthyl-phényl)-pipérazine et l'on prolonge le chauffage 8 heures supplémentaires. Après 28 heures, le mélange réactionnel est refroidi puis filtré. Le précipité est lavé par 2 fois 10 cm³ d'eau, 2 fois 5 cm³ d'éthanol, et 2 fois 5 cm³ de diisopropyl éther. On obtient 0,64 g de 8-chloro-7-fluoro-9-[4-(3-fluoro-4-méthyl-phényl)-pipérazin-1-yl]-4-méthyl-1-oxo-1,4-dihydro-benzo[f][1,7]naphtyridine-2-carboxylate d'éthyle sous forme d'un solide jaune verdâtre fondant à 350°C avec décomposition.

Le 8-chloro-7,9-difluoro-4-méthyl-1-oxo-1,4-dihydrobenzo[f][1,7]naphtyridine-2-carboxylate d'éthyle est obtenu de la façon suivante :
Une suspension agitée de 5,2 g de 8-chloro-7,9-difluoro-1-oxo-1,4-dihydrobenzo[f][1,7]naphtyridine-2-carboxylate d'éthyle, 4,15 g de carbonate de potassium anhydre dans 50 cm³ de diméthylformamide est chauffée à une température voisine de 110°C pendant 1 heure. Après refroidissement au voisinage de 50°C, on ajoute goutte à goutte 3,7 cm³ d'iodométhane. Le mélange est à nouveau porté à une température voisine de 100°C pendant 2 heures.
La masse réactionnelle est refroidie puis essorée. Le précipité est lavé par 3 fois 20 cm³ d'eau, 2 fois 15 cm³ d'éthanol, et 2 fois 15 cm³ de diéthyl éther. On obtient 4,7 g de 8-chloro-7,9-difluoro-4-méthyl-1-oxo-1,4-dihydro-benzo[f][1,7]naphtyridine-2-carboxylate d'éthyle, sous forme d'un solide beige fondant à 293°C.

Le 8-chloro-7,9-difluoro-1-oxo-1,4-dihydrobenzo[f][1,7]naphtyridine-2-carboxylate d'éthyle est obtenu de la façon suivante
Un mélange de 6,7 g de 7-chloro-6,8-difluoro-3-quinolylaminométhylènemalonate d'éthyle et de 70 cm³ de diphényl éther est chauffé à une température voisine de 240°C pendant 30 minutes. La masse réactionnelle est refroidie; le précipité est essoré, lavé par 5 fois 20 cm³ de diéthyl éther. On obtient 5,3 g de 8-chloro-7,9-difluoro-1-oxo-1,4-dihydro-benzo[f] [1,7] naphtyridine-2-carboxylate d'éthyle, sous forme d'un solide beige fondant à 328°C.

Le 7-chloro-6,8-difluoro-3-quinolylaminométhylènemalonate d'éthyle est obtenu de la façon suivante :
Un mélange de 4,25 g de 3-amino-7-chloro-6,8-difluoro-quinoléine et 4,4 cm³ d'éthoxyméthylènemalonate d'éthyle est chauffé à une température voisine de 120°C, sous agitation pendant 1 heure. La réaction est terminée par un chauffage supplémentaire de 15 minutes à une température voisine de 150°C.
La masse réactionnelle refroidie est reprise par 30 cm³ de diisopropyl éther, filtrée, lavée par 2 fois 15 cm³ de diisopropyl éther. On obtient 6,8 g de 7-chloro-6,8-difluoro-3-quinolylaminométhylènemalonate d'éthyle, sous forme d'un solide beige fondant à 175°C.

La 3-amino-7-chloro-6,8-difluoro-quinoléine est obtenue de la façon suivante :
4 g de 7-chloro-6,8-difluoro-3-nitro-quinoléine sont réduits dans 200 cm³ d'éthanol, en présence de 1 g environ de nickel de Raney, par l'hydrogène, à pression atmosphérique, à une température voisine de 20°C pendant 75 min.
Après addition de 100 cm³ de diméthylformamide à la masse réactionnelle, le catalyseur est filtré, la solution concentrée sous pression réduite (5 kPa) à une température voisine de 60°C. Le résidu est repris par 20 cm³ d'éthanol, essoré, lavé par 2 fois 15 cm³ de diéthyl éther. On obtient 3 g de 3-amino-7-chloro-6,8-difluoroquinoléine, sous forme d'un solide jaune fondant à 270°C.

Le 7-chloro-6,8-difluoro-3-nitroquinoléine est obtenu de la façon suivante :
A une solution de 8,7 g de 2-amino-4-chloro-3,5-difluoro-benzaldéhyde dans 300 cm³ d'éthanol, on ajoute rapidement, sous agitation, 17,4 g d'acide méthazonique et 60 cm³ d'une solution aqueuse d'acide chlorhydrique à 37%, en maintenant le mélange à une température voisine de 35°C. Le mélange reste agité 16 heures à une température voisine de 20°C. Le précipité est essoré, lavé avec 3 fois 25 cm³ de diéthyl éther. On obtient 8,1 g de 7-chloro-6,8-difluoro-3-nitro-quinoléine, sous forme d'un solide orange fondant à 174°C.

Le 2-amino-4-chloro-3,5-difluorobenzaldéhydeest obtenu de la façon suivante :
A une solution agitée de 13,4 g de 2-bromo-5-chloro-4,6-difluoro-aniline dans 150 cm³ de tétrahydrofurane (THF) anhydre refroidie à une température voisine de -75°C, on ajoute goutte à goutte 66 cm³ d'une solution 2N de butyllithium (dans l'hexane) en 30 minutes. Après agitation 1 heure à cette température, on ajoute 12,8 cm³ de diméthylformamide. Après l'addition, le mélange est encore agité 3 heures à une température voisine de -70°C. Le mélange est ramené à une température voisine de -5°C, puis on ajoute une solution aqueuse de chlorure d'ammonium (18 g dissous dans 180 cm³). Le mélange réactionnel est extrait par 2 fois 200 cm³ de diéthyl éther. Les extraits éthérés réunis sont lavés par 1 fois 150 cm³ d'eau et 1 fois 150 cm³ d'une solution aqueuse saturée de chlorure de sodium. Après séchage sur sulfate de magnésium, la solution est concentrée sous pression réduite (5 kPa), à une température voisine de 40°C. Après reprise du résidu solide obtenu dans 80 cm³ d'hexane, puis filtration, on obtient 6,2 g de 2-amino-4-chloro-3,5-difluorobenzaldéhyde, sous forme d'un solide jaune.

Le 2-bromo-5-chloro-4,6-difluoroaniline est obtenu de la façon suivante :
A une solution agitée de 17,4 g de 3-chloro-2,4-difluoro-aniline dans 150 cm³ de diméthylformamide sec, refroidie à une température voisine de -20°C, on ajoute graduellement 18,9 g de N-bromosuccinimide, en maintenant cette température. Après 1 heure d'agitation, on ramène la température au voisinage de 20°C, puis le mélange est concentré sous pression réduite (5 kPa), à une température voisine de 60°C. Le résidu obtenu est additionné de 400 cm³ d'hexane et de 200 cm³ d'eau. Le mélange est agité, la phase aqueuse décantée. Celle-ci est extraite par successivement 200, 200 et 100 cm³ d'hexane. Les extraits sont réunis, lavés par 2 fois 200 cm³ d'eau et 2 fois 200 cm³ d'une solution aqueuse saturée de chlorure de sodium. Après séchage sur sulfate de magnésium, la solution organique est concentrée sous pression réduite (5 kPa) à une température voisine de 40°C. On obtient 19,2 g de 2-bromo-5-chloro-4,6-difluoro-aniline, sous forme d'un solide blanc fondant à 62°C.

La 3-chloro-2,4-difluoroaniline est obtenue de la façon suivante :
A une suspension agitée de 23 g de 3-chloro-2,4-difluoro-nitrobenzène dans 110 cm³ de solution aqueuse d'acide chlorhydrique à 37% et 25 cm³ de diéthyl éther, on ajoute 135 g de chlorure stanneux (dihydrate) par petites fractions. Après l'addition, le mélange est chauffé pendant 30 minutes à une température voisine de 40°C. Après refroidissement de la masse réactionnelle, le mélange est versé sur 300 cm³ d'eau additionnée de 150 g de glace. Le mélange est rendu fortement alcalin par addition de lessive de soude, puis extrait par 2 fois 250 cm³ de chloroforme. Les extraits sont réunis, séchés sur sulfate de magnésium, puis concentrés sous pression réduite (5 kPa) à une température voisine de 40°C. On obtient 17,4 g de 3-chloro-2,4-difluoro-aniline, sous forme d'un solide beige fondant à 58°C.

### Exemple 2

L'acide-8-fluoro-4-méthyl-1-oxo-9-(1,3,3-triméthyl-6-azabicyclo[3.2.1]oct-6-yl)-1,4-dihydro-benzo[f][1,7]naphtyridine-2-carboxylique est préparé de la façon suivante :
Une suspension de 0,72 g de 8-fluoro-4-méthyl-1-oxo-9-( 1,3,3-triméthyl-6-azabicyclo[3.2.1]oct-6-yl)-1,4-dihydro-benzo[f][1,7]naphtyridine-2-carboxylate d'éthyle dans un mélange de 60 cm³ d'éthanol à 50% et 1,9 cm³ d'une solution aqueuse de potasse 1N est chauffée sous agitation pendant 3 heures à une température voisine de 70°C. Après concentration sous pression réduite (5,2 kPa) à 60°C, le résidu est dissous dans 75 cm³ d'eau. On élimine un très léger insoluble par filtration; la solution obtenue est neutralisée par 1,9 cm³ d'une solution aqueuse d'acide chlorhydrique 1N. L'insoluble formé est essoré , lavé par 3 fois 25 cm³ d'eau et séché sous vide à 100°C. On obtient 0,60 g d'acide-8-fluoro-4-méthyl-1-oxo-9-(1,3,3-triméthyl-6-azabicyclo[3.2.1]oct-6-yl)-1,4-dihydro-benzo[f][1,7]naphtyridine-2-carboxylique sous forme d'un solide jaune fondant à 200°C.

Le 8-fluoro-4-méthyl-1-oxo-9-( 1,3,3-triméthyl-6-azabicyclo[3.2.1]oct-6-yl)-1,4-dihydro-benzo[f][1,7]naphtyridine-2-carboxylate d'éthyle est obtenu de la façon suivante :
Une suspension de 2 g de 8,9-difluoro-4-méthyl-1-oxo-1,4-dihydro-benzo[f]-[1,7]-naphtyridine-2-carboxylate d'éthyle et de 2,6 g de 1,3,3-triméthyl-6-azabicyclo[3.2.1]-octane dans 50 cm³ de diméthylsulfoxyde est chauffée sous agitation à une température de 90°C pendant 12 jours. Après refroidissement à 20°C le mélange est versé dans 200 cm³ d'eau glacée . On extrait par 3 fois 200 cm³ de dichlorométhane, les extraits organiques sont lavés par 3 fois 100 cm³ d'eau et séchés sur sulfate de sodium. Après filtration et concentration sous pression réduite (5,2 kPa) le produit obtenu est purifié par chromatographie sur 160 g de gel de silice (0,06-0,20 mm). On élue en recueillant des fractions de 200 cm³ par 3 litres d'un mélange de dichlorométhane-éthanol (97-3) en volume. Les fractions comprises entre 1,65 et 3 litres sont concentrées à sec sous pression réduite (5,2 kPa). On obtient 0,9 g de 8-fluoro-4-méthyl-1-oxo-9-(1,3,3-triméthyl-6-azabicyclo[3.2.1]oct-6-yl)-1,4-dihydrobenzo[f][1,7]naphtyridine-2-carboxylate d'éthyle sous forme de cristaux jaunes fondant à 187°C.

Le 8,9-difluoro-4-méthyl-1-oxo-1,4-dihydro-benzo[f][1,7]naphtyridine-2-carboxylate d'éthyle est obtenu de la façon suivante :
Une suspension de 28,8 g de 8,9-difluoro-4-oxo-1,4-dihydrobenzo[f]-[1,7]naphtyridine-2-carboxylate d'éthyle et de 39 g de carbonate de potassium dans 450 cm³ de diméthylsulfoxyde est chauffée sous agitation à une température de 90°C pendant 3 heures. Après refroidissement à environ 50°C on ajoute par fractions 27 g d'iodure de méthyle. Le mélange est chauffé à 65°C pendant 3 heures; après refroidissement à 20°C on ajoute au mélange réactionnel 250 cm³ d'eau et 200 g de glace. L'insoluble est essoré , lavé par 3 fois 150 cm³ d'eau et dissous dans 2 litres de dichlorométhane. On décante l'eau résiduelle; les extraits organiques sont séchés sur sulfate de sodium en présence de noir animal. Après filtration et concentration sous pression réduite (5,2 kPa) le produit obtenu est purifié par chromatographie sur gel de silice (0,06-0,20 mm).On élue en recueillant des fractions de 250 cm³ par 6 litres d'un mélange de dichlorométhane-éthanol (97-3) en volume. Les fractions comprises entre 2 et 6 litres sont concentrées à sec sous pression réduite (5,2 kPa). On obtient 14 g de 8,9-difluoro-4-méthyl-1-oxo-1,4-dihydro-benzo[f][1,7]naphtyridine-2-carboxylate d'éthyle sous forme de cristaux blancs fondant à 272°C.

Le 8,9-difluoro-1-oxo-1,4-dihydro-benzo[f][1,7]naphtyridine-2-carboxylate d'éthyle est obtenu de la façon suivante :
Une suspension de 35,8 g de 6,7-difluoro-3-quinolylaminométhylènemalonate d'éthyle dans 250 cm³ de diphényl éther est chauffée sous agitation à une température de 230°C pendant 1 heure. On distille l'éthanol formé sous un courant d'argon. Après refroidissement à 50°C, on ajoute 500 cm³ d'hexane et on refroidit à 10°C. Le précipité obtenu est essoré et lavé par 3 fois 250 cm³ de diéthyl éther. On obtient 29 g de 8,9-difluoro-1-oxo-1,4-dihydro-benzo[f][1,7]naphtyridine-2-carboxylate d'éthyle sous forme de cristaux violets fondant à 300°C.

Le 6,7-difluoro-3-quinolylaminométhylènemalonate d'éthyle est obtenu de la façon suivante :
A une suspension de 19,2 g de 3-amino-6,7-difluoro-quinoléine dans 100 cm³ de toluène, on ajoute 25 g d'éthoxyméthylènemalonate d'éthyle. On chauffe sous agitation à une température de 110°C pendant 10 heures. On concentre à sec sous pression réduite (5,5 kPa) ; le produit isolé est repris sous agitation par 200 cm³ de diisopropyl éther, essoré et lavé par 2 fois 100 cm³ du même solvant. On obtient 35,9 g de 6,7-difluoro-3-quinolylaminométhylènemalonate d'éthyle sous forme de cristaux blancs fondant à 150°C.

Le 3-amino-6,7-difluoro-quinoléine est obtenu de la façon suivante :
A une solution aqueuse d'hypobromite de sodium à 0°C obtenue sous agitation par addition lente de 28,5 g de brome dans 500 cm³ d'une solution aqueuse de soude 2N, on ajoute sous agitation par fractions 33,2 g le 6,7-difluoroquinoléine-3-carboxamide. La solution verte obtenue est agitée pendant 3 heures à 0 °C et chauffée 3 heures à 60 °C.
   La suspension brune obtenue est refroidie à 0°C et acidifiée par 70 cm³ de solution aqueuse d'acide chlorhydrique 12N. L'insoluble est extrait 2 fois par 200 cm³ de diéthyl éther, les extraits aqueux acides neutralisés par une solution aqueuse d'ammoniaque à 33%. L'insoluble est essoré, lavé par 3 fois 150 cm³ d'eau et dissous dans 800 cm³ de diéthyl éther; les extraits organiques sont lavés par 3 fois 100 cm³ d'eau ,séchés sur sulfate de sodium en présence de 3 g de noir animal, filtrés et concentrés sous pression réduite (5,2 kPa). On obtient ainsi 20 g de 3-amino-6,7-difluoro-quinoléine sous forme de cristaux blancs fondant à 155°C.

Le 6,7-difluoroquinoléine-3-carboxamide est obtenu de la façon suivante :
A une suspension de 34 g d'acide 6,7-difluoro-quinoléine-3-carboxylique dans 350 cm³ de chloroforme, on ajoute 58 g de chlorure de thionyle et 3 gouttes de diméthylformamide. Le mélange est chauffé sous agitation à une température de 70°C pendant 4 heures et concentré sous pression réduite (5,2 kPa). Le produit blanc obtenu est mis en suspension dans 700 cm³ de dichlorométhane à une température de -10°C; en maintenant cette température, on fait barboter un courant d'ammoniac sous forte agitation. On laisse remonter la température du mélange à 20°C pendant 1 heure et concentre à sec sous pression réduite (5,2 kPa) à une température de 50°C. Le résidu blanc mis en suspension sous agitation dans 800 cm³ d'eau pendant 15 minutes est essoré et lavé par 3 fois 100 cm³ d'eau et 2 fois 100 cm³ d'éthanol. On obtient 33 g de 6,7-difluoro-quinoléine-3-carboxamide sous la forme de cristaux blancs fondant à 261°C.

L'acide 6,7-difluoro-quinoléine-3-carboxylique est obtenu de la façon suivante :
Une suspension de 37,7 g de 6,7-difluoroquinoléine-3-carboxylate d'éthyle dans un mélange de 20 cm³ d'éthanol et 160 cm³ d'une solution aqueuse de potasse 1N est agitée 4 heures à une température de 20°C. On additionne à la solution 600 cm³ d'eau, extrait l'insoluble par 2 fois 400 cm³ de diéthyl éther. La solution aqueuse est neutralisée par 161 cm³ d'une solution aqueuse d'acide chlorhydrique 1N. Le précipité obtenu est essoré, lavé par 3 fois 150 cm³ d'eau et séché sous vide à une température de 50°C. On obtient 30 g d'acide 6,7-difluoroquinoléine-3-carboxylique sous la forme de cristaux blancs fondant à 290°C.

Le 6,7-difluoroquinoléine-3-carboxylate d'éthyle est obtenu de la façon suivante :
Une solution de 52,9 g de 4-chloro-6,7-difluoroquinoléine-3-carboxylate d'éthyle dans 750 cm³ d'éthanol en présence de 19,7 g de triéthylamine est hydrogénée à pression atmosphérique, à une température voisine de 20 °C pendant 1 heure en présence de 3 g de palladium (type D) à 5% sur charbon. Après élimination du catalyseur par filtration à chaud, concentration à sec sous pression réduite (5,2 kPa) à environ 50°C, le résidu est repris sous agitation par 1,2 litre d'eau, essoré et lavé par 3 fois 200 cm³ d'eau. Ce dernier est dissous dans 1 litre de diéthyl éther; les extraits éthérés lavés avec 2 fois 100 cm³ d'eau sont séchés sur sulfate de sodium. Après filtration et concentration sous pression réduite (5,2 kPa) le produit obtenu est recristallisé dans 600 cm³ de diisopropyl éther. On obtient 42,8 g de 6,7-difluoroquinoléine-3-carboxylate d'éthyle sous la forme de cristaux blancs fondant à 135°C.

Le 4-chloro-6,7-difluoroquinoléine-3-carboxylate d'éthyle est obtenu de la façon suivante :
Une suspension de 58,3 g de 6,7-difluoro-4-oxo-quinoléine-3-carboxylate d'éthyle dans 490 g d'oxychlorure de phosphore est chauffée à 95°C pendant 5 heures sous agitation. Après évaporation à sec sous pression réduite (5,2 kPa) le résidu visqueux obtenu est additionné de 500 cm³ d'eau glacée et décomposé par addition lente d'une solution aqueuse saturée de carbonate de potassium jusqu'à pH 8. L'insoluble formé est extrait par 2 fois 400 cm³ de dichlorométhane; les extraits organiques obtenus sont séchés sur sulfate de sodium en présence de noir animal, filtrés et concentrés sous pression réduite (5,2 kPa). Le résidu obtenu est recristallisé dans 800 cm³ d'hexane. On obtient 53 g de 4-chloro-6,7-difluoroquinoléine-3-carboxylate d'éthyle sous forme de cristaux blancs fondant à 111°C.

Le 6,7-difluoro-4-oxo-quinoléine-3-carboxylate d'éthyle est préparé comme décrit dans Angeles de la Cruz et coll., Tetrahedron 48, 29, pp. 6135-6150 (1992)

### Exemple 3

L'acide 8-chloro-7-fluoro-9-[4-(3-chloro-4-fluoro-phényl)pipérazin-1-yl]-4-méthyl-1-oxo-1,4-dihydrobenzo[f][1,7]naphtyridine-2-carboxylique est obtenu de la façon suivante :
Une suspension agitée de 0,16 g de 8-chloro-7-fluoro-9-[4-(3-chloro-4-fluorophényl)-pipérazin-1-yl]-4-méthyl-1-oxo-1,4-dihydro-benzo[f] [1,7] naphtyridine-2-carboxylate d'éthyle dans 4 cm³ d'éthanol, 0,33 cm³ de solution aqueuse de potasse 1N et 2,5 cm³ d'eau distillée est chauffée au voisinage de 100°C pendant 3 heures. Le mélange obtenu est acidifié à cette même température par 0,5 cm³ d'acide acétique aqueux 1N.
Après refroidissement, l'insoluble est essoré, puis lavé par 3 fois avec 20 cm³ d'eau, 1 fois 10 cm³ d'éthanol et 3 fois 10 cm³ de diisopropyl éther. On obtient 0,12 g d'acide 8-chloro-7-fluoro-9-[4-(3-chloro-4-fluoro-phényl)-pipérazin-1-yl]-4-méthyl-1-oxo-1,4-dihydro-benzo[f] [1,7] naphtyridine-2-carboxylique, sous forme d'un solide jaune fondant à plus de 260°C.

Le 8-chloro-7-fluoro-9-[4-(3-chloro-4-fluoro-phényl)pipérazin-1-yl]-4-méthyl-1-oxo-1,4-dihydrobenzo[f][1,7]naphtyridine-2-carboxylate d'éthyle est obtenu de la façon suivante :
Une suspension agitée de 1,5 g de 8-chloro-7,9-difluoro-4-méthyl-1-oxo-1,4-dihydro-benzo[f] [1,7] naphtyridine-2-carboxylate d'éthyle et de 2,3 g de 1-(3-chloro-4-fluoro-phényl)-pipérazine dans 15 cm³ de diméthyllsulfoxide est chauffée à une température voisine de 100°C pendant 3 heures. Le milieu réactionnel est ensuite agité 15 heures à température ambiante puis on ajoute 10 cm³ d'éthanol et chauffe à une température voisine de 100°C pendant 21 heures. Le mélange réactionnel est concentré à sec sous pression réduite (5,2 kPa) à 80°C. Le résidu obtenu est repris par 50 cm³ d'éthanol; le précipité est filtré sur verre fritté, lavé par 3 fois 5 cm³ d'éthanol et séché . La poudre jaune obtenue est chromatographiée sur colonne de gel de silice Merck (granulométrie 0,04-0,06 mm, diamètre 2 cm, hauteur 20 cm), en éluant sous une pression de 50 kPa d'azote par un mélange de dichlorométhane et de méthanol (mélange 98/2 en volumes) et en recueillant des fractions de 50 cm³. Les fractions 9 à 16 sont réunies puis concentrées à sec sous pression réduite (2,7 kPa). Le solide jaune obtenu est lavé par 10 cm³ d'éthanol, filtré et séché. On obtient 0,21 g de 8-chloro-7-fluoro-9-[4-(3-chloro-4-fluoro-phényl)-pipérazine-1-yl]-4-méthyl-1-oxo-1,4-dihydrobenzo[f] [1,7] naphtyridine-2-carboxylate d'éthyle sous forme d'un solide jaune fondant à une température supérieure à 260°C.

Le 8-chloro-7,9-difluoro-4-méthyl-1-oxo-1,4-dihydrobenzo [f] [1,7] naphtyridine-2-carboxylate d'éthyle peut être préparé comme décrit à l'exemple 1.

La 4-(3-chloro-4-fluorophényl)-pipérazine est préparée de la façon suivante :
On chauffe à 90°C, sous un courant d'azote, pendant 24 heures un mélange de 7,5 g de 3-chloro-4-fluoro benzène, 3,9 g de tertiobutylate de sodium, 1,1 g de chlorure de 1,1'-bis(diphénylphosphino)ferrocényl palladium, 2,4 g de 1,1'-bis(diphénylphosphino)ferrocène et 300 cm³ de toluène. Le mélange réactionnel est refroidi à température ambiante et filtré sur verre fritté. Le filtrat est lavé par 2 fois 150 cm³ de dichlorométhane puis concentré à sec sous pression réduite (2,7 kPa). Le résidu obtenu est purifié par chromatographie sur colonne de gel de silice (granulométrie 0,04-0,06 mm, diamètre 7 cm, hauteur 30 cm) sous une pression de 50 kPa d'azote avec un mélange de dichlorométhane et de méthanol (80/20 en volumes) comme éluant et en recueillant des fractions de 150 cm³. Les fractions 22 à 29 sont réunies et concentrées à sec sous pression réduite (2,7 kPa). Le solide obtenu est repris par 20 cm³ de dichlorométhane et distillé au four à boule (180°C environ sous une pression de 0,5 mm de mercure). On obtient 2,3 g de 4-(3-chloro-4-fluorophényl)-pipérazine sous forme d'une huile incolore.
   Le chlorure de 1,1'-bis(diphénylphosphino)ferrocényl palladium peut être préparé comme décrit dans T. Hayashi et coll. publié dans J. Am. Chem. Soc., 1984, 106, p.158.

### Exemple 4

L'acide 8-fluoro-4-méthyl-1-oxo-7-[4-(3-trifluorométhyl-phényl)-pipérazin-1-yl]-1,4-dihydrobenzo[f][1,7]naphtyridine-2-carboxylique peut être préparé de la façon suivante :
Une suspension agitée de 0,40 g de 8-fluoro-4-méthyl-1-oxo-7-[4-(3-trifluorométhyl-phényl)-pipérazin-1-yl]-1,4-dihydrobenzo[f][1,7]naphtyridine-2 carboxylate d'éthyle dans 12 cm³ d'éthanol, 2,25 cm³ d'une solution aqueuse de potasse 1N et 4 cm³ d'eau est chauffée au voisinage de 100°C pendant 3 heures, puis agitée à température ambiante pendant 48 heures. La solution obtenue est acidifiée à cette même température par 2,5 cm³ d'une solution aqueuse d'acide chlorhydrique 1N.
Après refroidissement, l'insoluble est essoré, puis lavé par 5 fois avec 10 cm³ d'eau. On obtient 0,26 g d' acide 8-fluoro-4-méthyl-1-oxo-7-[4-(3-trifluorométhylphényl)-pipérazin-1-yl]-1,4-dihydrobenzo[f][1,7]naphtyridine-2-carboxylique sous forme d'un solide orange fondant à 261°C.

Le 8-fluoro-4-méthyl-1-oxo-7-[4-(3-trifluorométhyl-phényl)-pipérazin-1-yl]-1,4-dihydrobenzo[f][1,7]naphtyridine-2 carboxylate d'éthyle est obtenu de la façon suivante :
Une suspension agitée de 3 g de 7,8-difluoro-4-méthyl-1-oxo-1,4-dihydrobenzo[f] [1,7] naphtyridine-2-carboxylate d'éthyle et de 2,6 g de 4-(3-trifluorométhyl)-phényl pipérazine dans 30 cm³ de diméthylsulfoxyde est chauffée à une température voisine de 100°C pendant environ 100 heures. Le mélange réactionnel est traité avec 300 cm³ d'eau et extrait par 3 fois 100 cm³ de dichlorométhane. La phase organique est séchée sur sulfate de magnésium et concentrée à sec sous pression réduite (2,7 kPa). Le résidu obtenu est chromatographié sur colonne de gel de silice Merck (granulométrie 0,04-0,06 mm, diamètre 5 cm, hauteur 50 cm), en éluant sous une pression de 100 kPa d'argon par un mélange de dichlorométhane, d'acétate d'éthyle et de méthanol (mélange 49/49/2 en volumes) et en recueillant des fractions de 50 cm³. Les fractions 29 à 47 sont réunies, puis concentrées à sec sous pression réduite (2,7 kPa). On obtient 0,4 g de 8-fluoro-4-méthyl-1-oxo-7-[4-(3-trifluorométhylphényl)-pipérazin-1-yl]-1,4-dihydrobenzo[f][1,7]naphtyridine-2 carboxylate d'éthyle sous forme d'un solide jaune.

Le 7,8-difluoro-4-méthyl- 1-oxo-1,4-dihydro-benzo[f][1,7]naphtyridine-2-carboxylate d'éthyle peut être obtenu de la façon suivante :
Une suspension de 35 g de 7,8-difluoro-1-oxo-1,4-dihydrobenzo[f][1,7]naphtyridine-2-carboxylate d'éthyle et de 31,8 g de carbonate de potassium dans 350 cm³ de diméthylsulfoxyde est chauffée sous agitation à une température de 90°C pendant 3 heures. Après refroidissement à 50°C on ajoute 65 g d'iodure de méthyle; le mélange est chauffé à 90°C pendant 3 heures. Après refroidissement à 20°C, l'insoluble est essoré, lavé par 4 fois 100 cm³ d'eau, 3 fois 100 cm³ d'éthanol et 2 fois 100 cm³ de diéthyl éther. On obtient 25,5 g de la 7,8-difluoro-4-méthyl-1-oxo-1,4-dihydro-benzo[f][1,7]naphtyridine-2-carboxylate d'éthyle sous forme de cristaux blancs fondant à 320°C.

Le 7,8-difluoro-1-oxo-1,4-dihydro-benzo[f][1,7]naphtyridine-2-carboxylate d'éthyle peut être obtenu de la façon suivante :
Une suspension de 16 g de 7,8-difluoro-3-quinolylaminométhylènemalonate d'éthyle dans 110 cm³ de diphényl éther est chauffée sous agitation à une température de 235°C pendant 30 minutes. Après refroidissement à 50°C, on ajoute 250 cm³ d'hexane; le mélange est refroidi à 10°C. Le précipité obtenu est essoré et lavé par 3 fois 150 cm³ de diéthyl éther. On obtient 12,9 g d'un solide rosé fondant à 307°C.

Le 7,8-difluoro-3-quinolylaminométhylènemalonate d'éthyle peut être obtenu de la façon suivante :
A une suspension de 8,9 g de 3-amino-7,8-difluoro-quinoléine dans 50 cm³ de toluène on ajoute 11,5 g d'éthoxyméthylènemalonate d'éthyle. On chauffe sous agitation à une température de 110°C pendant 8. heures. On concentre à sec sous pression réduite (5,2 kPa); le produit isolé est repris sous agitation par 150 cm³ de diisopropyl éther, essoré et lavé par 2 fois 100 cm³ du même solvant. On obtient 16,1 g d'un produit sous forme de cristaux blancs fondant à 164°C.

La 3-amino-7,8-difluoro-quinoléine peut être obtenu de la façon suivante :
On traite 7,8 g de 7,8-difluoro-quinoléine-3-carboxamide avec une solution aqueuse d'hypobromite de sodium obtenue par addition de 6,4 g de brome à 118 cm³ d'une solution aqueuse de soude 2N. Le produit après traitement est dissous dans 600 cm³ de diéthyl éther; les extraits organiques sont lavés par 3 fois 50 cm³ d'eau puis séchés sur sulfate de sodium en présence de 2 g de noir animal. On isole 4,9 g de 3-amino-7,8-difluoro-quinoléine sous la forme de cristaux blancs fondant à 161°C.

La 7,8-difluoro-quinoléine-3-carboxamide peut être obtenue de la façon suivante :
A une suspension de 15,5 g d'acide 7,8-difluoro-quinoléine-3-carboxylique dans 100 cm³ de chloroforme on ajoute 51 g de chlorure de thionyle et 3 gouttes de diméthylformamide. Le produit isolé est mis en suspension dans 300 cm³ de dichlorométhane et traité comme à l'exemple 2. On obtient ainsi 14,8 g de 7,8-difluoro-quinoléine-3-carboxamide sous forme de cristaux blancs fondant à 250°C.

L'acide 7,8-difluoro-quinoléine-3-carboxylique peut être obtenu de la façon suivante
Une suspension de 17,6 g de 7,8-difluoroquinoléine-3-carboxylate diéthyle dans un mélange de 100 cm³ d'éthanol et de 90 cm³ de potasse 1N est agitée 1 heure à une température de 60°C. Après concentration sous pression réduite (5,2 kPa) le résidu est dissous dans 400 cm³ d'eau; l'insoluble est essoré et les extraits alcalins sont neutralisés par 91 cm³ de solution aqueuse d'acide chlorhydrique 1N. Le précipité obtenu par filtration est lavé par 3 fois 50 cm³ d'eau et séché sous vide à 50°C. On obtient 14,3 g d'acide 7,8-difluoro-quinoléine-3-carboxylique sous forme de cristaux blancs fondant à 275°C.

Le 7,8-difluoroquinoléine-3-carboxylate d'éthyle peut être obtenu de la façon suivante :
Une solution de 21,9 g de 4-chloro- 7,8-difluoroquinoléine-3-carboxylate d'éthyle dans 300 cm³ d'éthanol en présence de 8,8 g de triéthylamine et de 2,2 g de palladium à 5% sur charbon est hydrogénée à pression atmosphérique pendant 1 heure. Le résidu solide obtenu est recristallisé dans 300 cm³ d'hexane. On obtient 17,6 g de 7,8-difluoroquinoléine-3-carboxylate d'éthyle sous forme de cristaux blancs fondant à 118°C.

Le 4-chloro-7,8-difluoroquinoléine-3-carboxylate d'éthyle peut être préparé de la façon suivante :
Une suspension de 26 g de 7,8-difluoro-4-oxo-quinoléine-3-carboxylate d'éthyle dans 212 g d'oxychlorure de phosphore est chauffée à 95°C pendant 4 heures sous agitation. Après traitement, on obtient 27,3 g de 4-chloro-7,8-difluoroquinoléine-3-carboxylate d'éthyle sous la forme d'un solide blanc fondant à 114°C.

Le 7,8-difluoro-4-oxo-quinoléine-3-carboxylate d'éthyle peut être obtenu de la façon suivante :
Une suspension de 32,7 g de 2,3-difluorophénylaminométhylènemalonate d'éthyle dans 220 cm³ de diphényl éther est chauffée sous agitation à une température de 235°C pendant 2,5 heures. Après refroidissement à 50°C on ajoute 250 cm³ d'hexane. Le mélange est refroidi à 10°C; le précipité obtenu est essoré et lavé par 3 fois 200 cm³ de diéthyl éther. On obtient 26,2 g de 7,8-difluoro-4-oxo-quinoléine-3-carboxylate d'éthyle sous la forme d'un solide blanc fondant à 270°C.

Le 2,3-difluorophénylaminométhylènemalonate d'éthyle peut être obtenu de la façon suivante :
Un mélange de 15,1 g de 2,3-difluoro-aniline et de 26 g d'éthoxyméthylènemalonate d'éthyle est chauffé sous agitation à une température de 115°C pendant 2,5 heures. Après refroidissement le solide obtenu est recristallisé dans 200 cm³ d'hexane. On obtient 32,7 g de 2,3-difluorophénylaminométhylènemalonate d'éthyle sous la forme de cristaux blancs fondant à 97°C.

### Exemple 5

L'acide 9-(3,3-diméthylpipéridino)-8-fluoro-4-méthyl- 1-oxo-1,4-dihydrobenzo[f]-[1,7]-naphtyridine-2-carboxylique peut être préparé de la façon suivante :
A une suspension de 0,83 g de 9-(3,3-diméthyl-pipéridin-1-yl)-8-fluoro-4-méthyl-1-oxo-1,4-dihydro-benzo[f][1,7]naphtyridine-2-carboxylate d'éthyle dans 15 cm³ d'éthanol et 15 cm³ d'eau, sont ajoutés, sous agitation, 2,3 cm³ de potasse aqueuse 1N. Le mélange est chauffé 2 heures à environ 100°C. La solution est acidifiée par addition de 2,3 cm³ d'acide chlorhydrique aqueux 1N. Après refroidissement, le précipité est essoré, lavé par trois fois 10 cm³ d'eau, trois fois 10 cm³ d'éthanol et trois fois 10 cm³ de diéthyl éther. Le solide est séché à environ 50°C sous pression de 1 kPa pendant 4 heures. On obtient 0,75 g d'acide 9-(3,3-diméthyl-pipéridin-1-yl)-8-fluoro-4-méthyl-1-oxo-1,4-dihydrobenzo[f][1,7]naphtyridine-2-carboxylique sous forme d'un solide jaune fondant vers 292-295°C.

Le 9-(3,3-diméthylpipéridino)-8-fluoro-4-méthyl-1-oxo-1,4-dihydrobenzo[f][1,7]-naphtyridine-2-carboxylate d'éthyle et le 8-(3,3-Diméthylpipéridino)-9-fluoro-4-méthyl-1-oxo-1,4-dihydrobenzo[f][1,7]naphtyridine-2-carboxylate d'éthyle peuvent être obtenu de la façon suivante :
Une suspension de 2 g de 8-9-difluoro-4-méthyl-1-oxo-1,4-dihydrobenzo[f][1,7]naphtyridine-2-carboxylate d'éthyle et de 3,23 cm³ de 3,3-diméthylpipéridine dans 40 cm³ de diméthylsulfoxyde est chauffée à une température voisine de 80°C pendant 72 heures. Après refroidissement à environ 20°C, 200 cm³ d'eau sont ajoutés au mélange réactionnel. Le précipité obtenu est essoré et lavé par 25 cm³ d'eau. Le solide est repris par 100 cm³ de dichlorométhane, l'eau résiduelle décantée; la solution organique est séchée sur sulfate de magnésium et concentrée sous pression réduite (5 kPa) à environ 40°C. On obtient 2,67 g d'un mélange contenant le 9-(3,3-diméthyl-pipéridin-1-yl)-8-fluoro-4-méthyl-1-oxo-1,4-dihydrobenzo[f][1,7]naphtyridine-2-carboxylate d'éthyle et le 8-(3,3-diméthyl-pipéridin-1-yl)-9-fluoro-4-méthyl-1-oxo-1,4-dihydro-benzo[f][1,7]naphtyridine-2-carboxylate d'éthyle. Ce mélange est purifié par flash chromatographie sur gel de silice (granulométrie 20-45 µm, diamètre 10 cm, hauteur 60 cm ) sous pression de 80 kPa en éluant par un mélange à 99% de dichlorométhane et 1% de méthanol et en fractionnant par 70 cm³. Après concentration des fractions 16 à 39 sous pression réduite (5 kPa) à environ 50°C, on obtient 0,85 g de 9-(3,3-diméthyl-pipéridin-1-yl)-8-fluoro-4-méthyl-1-oxo-1,4-dihydro-benzo[f][1,7]naphtyridine-2-carboxylate d'éthyle sous forme d'un solide jaune fondant vers 218-220°C.
   Après concentration sous pression réduite (5 kPa) à environ 50°C des fractions 104 à 216, on obtient 0,5 g de 8-(3,3-diméthyl-pipéridin-1-yl)-9-fluoro-4-méthyl-1-oxo-1,4-dihydro-benzo[f][1,7]naphtyridine-2-carboxylate d'éthyle sous forme d'un solide jaune fondant à environ 198-200°C.

Le 8-9-difluoro-4-méthyl-1-oxo-1,4-dihydrobenzo[f][1,7]naphtyridine-2-carboxylate d'éthyle peut être obtenu comme décrit à l'exemple 2.

### Exemple 6

L'acide 8-(-3,3-diméthylpipéridino)-9-fluoro-4-méthyl-1-oxo-1,4-dihydrobenzo[f]-[1,7]naphtyridine-2-carboxylique peut être préparé de la façon suivante :
A une suspension de 0,5 g de 8-(3,3-diméthyl-pipéridin-1-yl)-9-fluoro-4-méthyl-1-oxo-1,4-dihydro-benzo[f][1,7]naphtyridine-2-carboxylate d'éthyle dans 15 cm³ d'éthanol et 15 cm³ d'eau, sont ajoutés, sous agitation, 1,4 cm³ de potasse aqueuse 1N. Le mélange est porté à environ 100°C pendant une heure. La suspension obtenue est acidifiée par addition de 1,4 cm³ d'acide chlorhydrique aqueux 1N. Après refroidissement, le précipité est essoré, lavé par trois fois 10 cm³ d'eau, trois fois 10 cm³ d'éthanol et trois fois 10 cm³ d'éther. Le solide est séché à environ 80°C sous 1 kPa pendant 4 heures. On obtient 0,38 g d'acide 8-(-3,3-diméthyl-pipéridin-1-yl)-9-fluoro-4-méthyl-1-oxo-1,4-dihydro-benzo[f][1,7]naphtyridine-2-carboxylique sous forme d'un solide jaune fondant avec décomposition à environ 286-7°C.

La préparation du 8-(3,3-diméthyl-pipéridin-1-yl)-9-fluoro-4-méthyl-1-oxo-1,4-dihydro-benzo[f][1,7]naphtyridine-2-carboxylate d'éthyle a été décrite dans l'exemple 5.

### Exemple 7

L'acide-7-fluoro-8-[4-(3-fluoro-4-méthyl-phényl)-pipérazin-1-yl]-4-méthyl-1-oxo-1,4-dihydrobenzo[f][1,7] naphtyridine-2-carboxylique peut être obtenu de la façon suivante :
Une suspension agitée de 1,0 g de 7-fluoro-8-[4-(3-fluoro-4-méthyl-phényl)-pipérazin-1-yl]-4-méthyl-1-oxo-1,4-dihydro-benzo[f] [1,7] naphtyridine-2-carboxylate d'éthyle dans 20 cm³ d'acide acétique à 100% et 2 cm³ d'acide chlorhydrique à 37% est chauffée au voisinage de 110°C pendant 7 heures. La solution obtenue est concentrée sous pression réduite (20 kPa), le résidu est repris par trois fois 30 cm³ d'eau à environ 80°C.
L'insoluble est essoré, puis lavé par 3 fois avec 30 cm³ d'eau, 3 fois avec 30 cm³ d'éthanol et enfin 3 fois avec 20 cm³ de diéthyl éther. On obtient 0,79 g d'acide-7-fluoro-8-[4-(3-fluoro-4-méthyl-phényl)-pipérazine-1-yl]-4-méthyl-1-oxo-1,4-dihydrobenzo[f] [1,7] naphtyridine-2-carboxylique, sous forme d'un solide jaune fondant à plus de 300°C.

Le 7-fluoro-8-[4-(3-fluoro-4-méthyl-phényl)-pipérazine-1-yl]-4-méthyl-1-oxo-1,4-dihydrobenzo[f][1,7] naphtyridine-2-carboxylate d'éthyle peut être préparé comme suit:
Une suspension agitée de 3 g de 7,8-difluoro-4-méthyl-1-oxo-1,4-dihydrobenzo[f][1,7] naphtyridine-2-carboxylate d'éthyle et de 3,9 g de 1-(3-fluoro-4-méthylphényl)-pipérazine dans 30 cm³ de diméthylsulfoxyde est chauffée à une température voisine de 100°C pendant environ 100 heures. Le mélange réactionnel est traité avec 150 cm³ d'eau aux environs de 10°C et le précipité est repris par 100 cm³ de dichlorométhane, séché avec 10 g de sulfate de magnésium et concentré à sec sous pression réduite (20 kPa). La poudre jaune obtenue est chromatographiée sur colonne de gel de silice Merck (granulométrie 0,04-0,06 mm, diamètre 3 cm, hauteur 80 cm), en éluant sous une pression de 1 bar d'argon par un mélange de dichlorométhane et de méthanol (mélange 98/2 en volumes) et en recueillant des fractions de 200 cm³. Les fractions 16 à 23 sont réunies, puis concentrées à sec sous pression réduite (2,7 kPa). Le solide jaune obtenu est lavé par 10 cm³ de diéthyl éther, filtré et séché. On obtient 1,1 g de-7-fluoro-8-[4-(3-fluoro-4-méthyl-phényl)-pipérazine-1-yl]-4-méthyl-1-oxo-1,4-dihydro-benzo[f] [1,7] naphtyridine-2-carboxylate d'éthyle sous forme d'un solide jaune fondant à une température voisine de 290°C.

Le 7,8-difluoro-4-méthyl-1-oxo-1,4-dihydrobenzo[f][1,7]naphtyridine-2-carboxylate d'éthyle est préparé comme décrit à l'exemple 4.

La 1-(3-fluoro-4-méthyl-phényl)-pipérazine est préparée selon la méthode décrite dans les brevets DE 1019308 et US 2,830,056.

### Exemple 8

L'acide 8-fluoro-7-[4-hydroxy-4-(3-trifluorométhyl-phényl)-pipéridin-1-yl]-4-méthyl-1-oxo-1,4-dihydro-benzo[f][1,7]naphtyridine-2-carboxylique peut être préparé de la façon suivante :
A une suspension de 0,46 g de 8-fluoro-7-[4-hydroxy-4-(3-trifluorométhylphényl)-pipéridin-1-yl]-4-méthyl-1-oxo-1,4-dihydro-benzo[f][1,7]naphtyridine-2-carboxylate d'éthyle dans 5 cm³ d'éthanol et 4 cm³ d'eau, sont ajoutés, sous agitation, 1,02 cm³ de potasse aqueuse 1N. Le mélange est porté à environ 80°C pendant 2 heures puis évaporé à sec. Le résidu est dissous dans 20 cm³ d'eau, la phase aqueuse est lavée par 20 cm³ de dichlorométhane, acidifiée par 0,06 cm³ d'acide acétique, puis extraite par 3 fois 50 cm³ de dichlorométhane. La phase organique est séchée sur sulfate de magnésium et concentrée à sec sous pression réduite (20 kPa). Le résidu obtenu est lavé 2 fois par 5 cm³ de diisopropyl éther et séché. On obtient 0,22 g d'acide 8-fluoro-7-[4-hydroxy-4-(3-trifluorométhyl-phényl)-pipéridin-1-yl]-4-méthyll-oxo-1,4-dihydro-benzo[f][1,7]naphthyridine-2-carboxylique sous forme d'un solide jaune fondant à 245°C.

Le 8-fluoro-7-[4-hydroxy-4-(3-trifluorométhyl-phényl)-pipéridin-1-yl]-4-méthyl-1-oxo-1,4-dihydro-benzo[f][1,7]naphtyridine-2-carboxylate d'éthyle et le 7-fluoro-8-[4-hydroxy-4-(3-trifluorométhyl-phényl)-pipéridin-1-yl]-4-méthyl-1-oxo-1,4-dihydrobenzo[f][1,7]naphtyridine-2-carboxylate d'éthyle peuvent être obtenus de la façon suivante :
Une suspension de 2,23 g de 7-8-difluoro-4-méthyl-1-oxo-1,4-dihydrobenzo[f][1,7]naphtyridine-2-carboxylate d'éthyle, de 3 g de chlorhydrate de 4-hydroxy-4-(3-trifluorométhyl-phényl)-pipéridine et de 3 cm³ de triéthylamine dans 20 cm³ de diméthylsulfoxyde est chauffée à une température voisine de 100°C pendant 24 heures, puis on ajoute à nouveau 3 g de chlorhydrate de 4-hydroxy-4-(3-trifluorométhyl-phényl)-pipéridine et 3 cm³ de triéthylamine dans 5 cm³ de diméthylsulfoxyde et on chauffe à une température voisine de 100°C pendant 72 heures . Après refroidissement à environ 20°C, le milieu réactionnel est filtré, le filtrat est traité par 100 g de glace pilée puis 100 cm³ d'eau, puis extrait par 200 cm³ de dichlorométhane. La phase organique est séchée sur sulfate de magnésium et concentrée sous pression réduite (5 kPa) à environ 40°C. On obtient 3,93 g d'un mélange de 8-Fluoro-7-[4-hydroxy-4-(3-trifluorométhyl-phényl)-pipéridin-1-yl]-4-méthyl-1-oxo-1,4-dihydro-benzo[f][1,7]naphtyridine-2-carboxylate d'éthyle et de 7-fluoro-8-[4-hydroxy-4-(3-trifluorométhyl-phényl)-pipéridin-1-yl]-4-méthyl-1-oxo-1,4-dihydro-benzo[f][1,7]naphtyridine-2-carboxylate d'éthyle. Ce mélange est purifié par flash chromatographie sur gel de silice (granulométrie 20-45 µm, diamètre 5,1 cm, poids de silice 393 g) sous 50 kPa d'argon en éluant par un mélange à 98% de dichlorométhane et 2% d'éthanol et en fractionnant par 50 cm³. Après concentration des fractions 55 à 66 sous pression réduite (5 kPa) à environ 50°C, on obtient 0,34 g de 8-fluoro-7-[4-hydroxy-4-(3-trifluorométhyl-phényl)-pipéridin-1-yl]-4-méthyl-1-oxo-1,4-dihydro-benzo[f][1,7]naphtyridine-2-carboxylate d'éthyle sous forme d'un solide jaune fondant vers 122°C.
Après concentration sous pression réduite (5 kPa) à environ 50°C des fractions 112 à 149, on obtient 0,3 g de 7-fluoro-8-[4-hydroxy-4-(3-trifluorométhyl-phényl)-pipéridin-1-yl]-4-méthyl-1-oxo-1,4-dihydro-benzo[f][1,7]naphtyridine-2-carboxylate d'éthyle sous forme d'un solide jaune fondant à plus de 260°C.

Le 7-8-difluoro-4-méthyl- 1-oxo-1,4-dihydrobenzo[f][1,7]naphtyridine-2-carboxylate d'éthyle est obtenu comme décrit dans l'exemple 4.

### Exemple 9

L'acide 7-fluoro-8-[4-hydroxy-4-(3-trifluorométhyl-phényl)-pipéridin-1-yl]-4-méthyl-1-oxo-1,4-dihydro-benzo[f][1,7]naphtyridine-2-carboxylique peut être préparé de la façon suivante :
A une suspension de 0,39 g de 7-fluoro-8-[4-hydroxy-4-(3-trifluorométhylphényl)-pipéridin-1-yl]-4-méthyl-1-oxo-1,4-dihydro-benzo[f][1,7]naphtyridine-2-carboxylate d'éthyle dans 4,5 cm³ d'éthanol et 3,5 cm³ d'eau, sont ajoutés, sous agitation, 0,86 cm³ de potasse aqueuse 1N. Le mélange est porté à environ 80°C pendant 3 heures, puis après refroidissement on ajoute 20 cm³ d'eau, puis on acidifie par 0,05 cm³ d'acide acétique. Le précipité est filtré, essoré, lavé par 2 cm³ d'isopropanol puis 2 fois par 2 cm³ de diisopropyl éther et séché. On obtient 0,25 g d'acide 7-fluoro-8-[4-hydroxy-4-(3-trifluorométhyl-phényl)-pipéridin-1-yl]-4-méthyl-1-oxo-1,4-dihydro-benzo[f][1,7]naphtyridine-2-carboxylique sous forme d'un solide jaune fondant au dessus de 260°C.

Le 7-fluoro-8-[4-hydroxy-4-(3-trifluorométhyl-phényl)-pipéridin-1-yl]-4-méthyl-1-oxo-1,4-dihydro-benzo[f][1,7]naphtyridine-2-carboxylate d'éthyle peut être obtenu comme décrit dans l'exemple 8.

### Exemple 10

L'acide 9-[2-anilinométhyl-(2S)-pyrrolidino]-8-fluoro-4-méthyl-1-oxo-1,4-dihydrobenzo[f][1,7]naphtyridine-2-carboxylique peut être préparé de la façon suivante :
A une suspension de 0,66 g de (S)-8-fluoro-4-méthyl-1-oxo-9-(2-phénylaminométhyl-pyrrolidin-1-yl)-1,4-dihydro-benzo[f][1,7]naphtyridine-2-carboxylate d'éthyle dans 15 cm³ d'éthanol et 10 cm³ d'eau, sont ajoutés, sous agitation, 3,4 cm³ de potasse aqueuse 1N. Le mélange est porté à environ 100°C pendant 24 heures. La suspension obtenue est acidifiée par addition de 3,4 cm³ d'acide chlorhydrique aqueux 1N. Après refroidissement, le solide est essoré et lavé par trois fois 50 cm³ d'eau et séché à environ 100°C pendant 4 heures sous pression del kPa. On obtient 1 g d'acide (S)-8-fluoro-4-méthyl-1-oxo-9-(2-phénylaminométhylpyrrolidin-1-yl)-1,4-dihydro-benzo[f][1,7]naphtyridine-2-carboxylique sous forme d'un solide orange fondant vers 250°C.

Le 9-[2-anilinométhyl-(2S)-pyrrolidino]-8-fluoro-4-méthyl- 1-oxo-1,4-dihydrobenzo[f][1,7]naphtyridine-2-carboxylate d'éthyle peut être obtenu de la façon suivante :
Une suspension de 2,4 g de 8-9-difluoro-4-méthyl-1-oxo-1,4-dihydrobenzo[f][1,7]naphtyridine-2-carboxylate d'éthyle et de 3,25 g de (S)-2-(phénylaminométhyl)-pyrrolidine dans 50 cm³ de diméthylsulfoxyde est chauffée à une température voisine de 80°C pendant 96 heures. Après refroidissement à environ 20°C, 200 cm³ d'eau sont ajoutés au mélange réactionnel. Le précipité obtenu est essoré et lavé par 25 cm³ d'eau. Le solide (3,4 g) est purifié par flash chromatographie sur gel de silice (granulométrie 20-45µm, diamètre 4cm, hauteur 40 cm) sous 80 kPa en éluant par un mélange à 99% de dichlorométhane et 1% d'éthanol et en fractionnant par 30 cm³. Après concentration des fractions 40 à 60 sous pression réduite (5 kPa) à environ 50°C, on obtient 1,5 g d'un solide qui après une recristallisation dans 100 cm³ d'isopropanol, produit 1,3 g de (S)-8-fluoro-4-méthyl-1-oxo-9-(2-phénylaminométhyl-pyrrolidin-1-yl)-1,4-dihydro-benzo[f][1,7]naphtyridine-2-carboxylate d'éthyle sous forme d'un solide jaune fondant vers 170°C.

Le 8-9-difluoro-4-méthyl-1-oxo-1,4-dihydrobenzo[f][1,7]naphtyridine-2-carboxylate d'éthyle peut être obtenu comme décrit à l'exemple 2.

### Exemple 11

L'acide 9-[3-(4-benzyloxy-3-méthoxy-phényl)-pipérazin-1 -yl]-8-fluoro-4-méthyl-1-oxo-1,4-dihydrobenzo[f][1,7]-naphtyridine-2-carboxylique peut être préparé de la façon suivante :
Une suspension agitée de 0,68 g de 9-[3-(4-benzyloxy-3-méthoxy-phényl)-4-trifluorométhylacétyl-piperazin-1-yl]-8-fluoro-4-méthyl-1-oxo-1,4-dihydrobenzo[f][1,7]naphtyridine-2-carboxylate d'éthyle dans 6,8 cm³ d'eau, 20,4 cm³ d'éthanol et 2,95 cm³ de potasse aqueuse 1N est chauffée au voisinage de 100°C pendant 4heures. L'éthanol est chassé sous pression réduite (5 kPa) à environ 50°C, on ajoute à la solution obtenue 100 cm³ d'eau, on filtre puis le filtrat est acidifié par 2 cm³ d'une solution aqueuse d'acide chlorhydrique 1N.
Après chauffage 1 heure à 65 °C puis refroidissement, l'insoluble est essoré, puis lavé par 3 fois avec 20 cm³ d'eau, 3 fois 20 cm³ d'éthanol, 3 fois 30 cm³ de pentane. Après séchage, on obtient 0,46 g d'acide 9-[3-(4-benzyloxy-3-méthoxy-phényl)-pipérazin-1-yl]-8-fluoro-4-méthyl-1-oxo-1,4-dihydro-benzo[f][1,7]naphtyridine-2-carboxylique, sous forme d'un solide fondant à 280°C.

Le 9-[3-(4-Benzyloxy-3-méthoxy-phényl)-4-trifluorométhylacétyl-pipérazin-1-yl]-8-fluoro-4-méthyl-1-oxo-1,4-dihydro-benzo[f][1,7]naphtyridine-2-carboxylate d'éthyle peut être obtenu de la façon suivante :
Une suspension de 2,38 g de 8,9-difluoro-4-méthyl-1-oxo-1,4-dihydro-benzo[f] [1,7] naphtyridine-2-carboxylate d'éthyle, de 2,23 g de 2-(4-benzyloxy-3-méthoxyphényl)-pipérazine, et de 12,6 cm³ de triéthylamine dans 50cm³ de diméthylsulfoxyde est chauffée à une température voisine de 90°C pendant 170 heures.
Le mélange refroidi est versé sur 500 cm³ d'eau glacée, sous agitation. L'insoluble est extrait par 5 fois 100 cm³ de dichlorométhane. La phase organique est séchée sur sulfate de magnésium, concentrée sous pression réduite (5 kPa) à environ 40°C. Le résidu obtenu (3,66 g) est dissous dans 70 cm³ de dichlorométhane, on ajoute à cette solution 4,3 cm³ d'anhydride trifluoroacétique et le mélange réactionnel est agité 24 heures à température ambiante. On ajoute ensuite 5 g d'hydrogénocarbonate de sodium, 100 cm³ d'eau et on extrait avec du dichlorométhane. Les phases organiques sont réunies puis après séchage sur sulfate de magnésium on concentre sous pression réduite (5 kPa) à environ 40°C. Le résidu obtenu est purifié trois fois par chromatographie sur une colonne de gel de silice (granulométrie 0,04-0,063; diamètre 6 cm, hauteur 40 cm) sous 50 kPa d'argon, en éluant par un mélange de dichlorométhane et d'éthanol (99/1 en volumes) . Après concentration à sec sous pression réduite (5 kPa) à environ 40°C des fractions les moins polaires, on obtient 0,69 g de 9-[3-(4-benzyloxy-3-méthoxy-phényl)-4-trifluorométhylacétyl-pipérazin-1-yl]-8-fluoro-4-méthyl-1-oxo-1,4-dihydrobenzo[f]-[1,7]naphtyridine-2-carboxylate d'éthyle sous forme d'un solide jaune fondant à 154°C.

Le 8,9-difluoro-4-méthyl-1-oxo-1,4-dihydrobenzo[f][1,7]naphtyridine-2-carboxylate d'éthyle peut être obtenu comme décrit dans l'exemple 2.

### Exemple 12

L'acide 8-fluoro-9-[4-hydroxy-4-(3-trifluorométhylphényl)pipéridino]-4-méthyl-1-oxo-1,4-dihydrobenzo[f]-[1,7]naphtyridine-2-carboxylique peut être préparé de la façon suivante :
Une suspension agitée de 1,09 g de 8-fluoro-9-[4-hydroxy-4-(3-trifluorométhylphényl)-pipéridine-1-yl]-4-méthyl-1-oxo-1,4-dihydro-benzo[f] [1,7] naphtyridine-2-carboxylate d'éthyle dans 8 cm³ d'eau, 10 cm³ d'éthanol et 2,4 cm³ d'une solution aqueuse de potasse 1N est chauffée au voisinage de 100°C pendant 1 heure et 30 minutes. La solution obtenue est acidifiée à cette même température par 2,5 cm³ d'une solution aqueuse d'acide acétique 1N.
Après refroidissement, l'insoluble est essoré, puis lavé par 2 fois avec 10 cm³ d'eau, 1 fois 5 cm³ d'éthanol, 2 fois 5 cm³ de diéthyl éther. Après une recristallisation dans 5 cm³ de diméthylformamide, on obtient 0,72 g d'acide 8-fluoro-9-[4-hydroxy-4-(3-trifluorométhyl-phényl)-pipéridine- 1-yl]-4-méthyl-1-oxo-1,4-dihydro-benzo[f] [1,7] naphtyridine-2-carboxylique, sous forme d'un solide jaune fondant à 300°C.

Le 8-fluoro-9-[4-hydroxy-4-(3 -trifluorométhylphényl)pipéridino]-4-méthyl-1-oxo-1,4-dihydrobenzo[f][1,7]naphtyridine-2-carboxylate d'éthyle peut être obtenu de la façon suivante :
Une suspension de 3,7 g de chlorhydrate de 4[3-(trifluorométhyl)phényl]-4 pipéridinol dans 50 cm³ de diméthylsulfoxyde est agitée à une température voisine de 25°C avec 2,8 cm³ de triéthylamine, pendant 10 minutes. Après addition de 2 g de 8,9-difluoro-4-méthyl-1-oxo-1,4-dihydro-benzo[fl [1,7] naphtyridine-2-carboxylate d'éthyle, on chauffe le mélange à 100°C pendant 170 heures.
Le mélange refroidi est versé sur environ 150 cm³ d'eau glacée, sous agitation. L'insoluble est extrait par respectivement 200, 100, 50 cm³ de dichlorométhane Après refroidissement des phases organiques, le précipité obtenu est filtré. Le filtrat est lavé par 2 fois 200cm³ d'eau, puis séché sur sulfate de magnésium, concentré sous pression réduite (5 kPa) à environ 40°C. Le résidu obtenu est purifié par chromatographie sur une colonne de gel de silice (granulométrie 0,04-0,063; diamètre 6 cm, hauteur 40 cm) à pression atmosphérique, en éluant par un mélange de dichlorométhane et d'éthanol (95/5 en volumes) et en recueillant des fractions de 25 cm³. Les fractions 45 à 60 sont concentrées à sec sous pression réduite (5 kPa) à environ 40°C. Après lavage par 20 cm³ de diéthyl éther, et essorage, on obtient 1,10 g de 8-fluoro-9-[4-hydroxy-4-(3-trifluorométhyl-phényl)-pipéridine-1-yl]-4-méthyl-1-oxo-1,4-dihydro-benzo[f] [1,7] naphtyridine-2-carboxylate d'éthyle, sous forme d'un solide jaune fondant à 262°C.

Le 8,9-difluoro-4-méthyl-1-oxo-1,4-dihydrobenzo[f][1,7]naphtyridine-2-carboxylate d'éthyle peut être obtenu comme décrit dans l'exemple 2.

### Exemple 13

L'acide 8-fluoro-9-[4-(3-méthoxyphényl)-3-méthylpipérazin-1-yl]-4-méthyl-1-oxo-1,4-dihydrobenzo[f][1,7]naphtyridine-2-carboxylique peut être préparé de la façon suivante :
A une suspension de 1 g de 8-fluoro-9-[4-(3-méthoxy-phényl)-3-méthylpipérazine-1-yl]-4-méthyl-1-oxo-1,4-dihydro-benzo[f][1,7]naphtyridine-2-carboxylate d'éthyle dans 10 cm³ d'éthanol et 20 cm³ d'eau chauffée à une température voisine de 50°C, sont ajoutés, sous agitation, 2,4 cm³ de potasse aqueuse 1N. Le mélange est porté au voisinage de 100°C pendant 2heures et 30 minutes.
La solution obtenue est acidifiée à 100°C par 2,5 cm³ d'une solution aqueuse d'acide chlorhydrique 1N. Après refroidissement, l'insoluble est essoré puis lavé par 2 fois avec 10 cm³ d'eau. Après dissolution du solide obtenu dans environ 150 cm³ de dichlorométhane, puis décantation de l'eau résiduelle, la solution est séchée sur sulfate de magnésium, puis concentrée sous pression réduite (5 kPa) à environ 40°C. Le solide obtenu est repris dans environ 30 cm³ de diéthyl éthér, essoré, séché à l'air. On obtient 0,88 g d'acide 8-fluoro-9-[4-(3-méthoxy-phényl)-3-méthyl-pipérazine-1-yl]-4-méthyl-1-oxo-1,4-dihydro-benzo[f] [1,7] naphtyridine-2-carboxylique, sous forme d'un solide jaune fondant à 285°C.

Le 8-Fluoro-9-[4-(3-méthoxyphényl)-3-méthylpipérazin-1-yl]-4-méthyl-1-oxo-1,4-dihydrobenzo[f][1,7]naphtyridine-2-carboxylate d'éthyle peut être obtenu de la façon suivante :
Une suspension de 2 g de 8,9-difluoro-4-méthyl-1-oxo-1,4-dihydro-benzo[f] [1,7] naphtyridine-2-carboxylate d'éthyle, et de 2,85 g de 1-(3-méthoxy-phényl)-2-méthyl-pipérazine dans 50 cm³ de diméthylsulfoxyde est chauffée sous agitation, à une température voisine de 100°C. Au bout de 230 heures, on ajoute 1,45 g supplémentaires de 1-(3-méthoxy-phényl)-2-méthyl-pipérazine.
Après 170 heures de chauffage, le mélange est refroidi puis versé sur environ 200 cm³ d'eau glacée. L'insoluble est essoré, lavé 2 fois avec 20 cm³ d'eau. Après dissolution du solide obtenu dans 50 cm³ de dichlorométhane, la solution obtenue est lavée une fois avec 25 cm³ d'eau, décantée, séchée sur sulfate de magnésium, puis concentrée sous pression réduite (5 kPa) à environ 40°C. Le résidu obtenu est purifié par chromatographie sur une colonne de gel de silice (granulométrie 0,04-0,063mm; diamètre 6 cm, hauteur 50 cm) à pression atmosphérique, en éluant par un mélange de dichlorométhane et d'éthanol (98/2 en volumes) et en recueillant des fractions de 30 cm³. Les fractions 50 à 70 sont concentrées à sec sous pression réduite (5 kPa) à environ 40°C. On obtient 1 g de 8-fluoro-9-[4-(3-méthoxy-phényl)-3-méthylpipérazine-1-yl]-4-méthyl-1-oxo-1,4-dihydro-benzo[f][1,7]naphtyridine-2-carboxylate d'éthyle sous forme d'un solide jaune fondant à 226°C.

Le 8,9-difluoro-4-méthyl-1-oxo-1,4-dihydrobenzo[f][1,7]naphtyridine-2-carboxylate d'éthyle peut être obtenu comme décrit dans l'exemple 2.

### Exemple 14

L'acide 9-[3-(3,4-diméthyl-phényl)-pipérazin-1-yl]-8-fluoro-4-méthyl-1-oxo-1,4-dihydrobenzo[f][1,7]naphthyridine-2-carboxylique peut être préparé de la façon suivante :
Une suspension agitée de 1,47 g de 9-[3-(3,4-diméthyl-phényl)-pipérazin-1-yl]-8-fluoro-4-méthyl-1-oxo-1,4-dihydro-benzo[f][1,7]naphthyridine-2-carboxylate d'éthyle dans 25 cm³ d'éthanol, 3,60 cm³ de potasse aqueuse 1N et 15 cm³ d'eau est chauffée au voisinage de 100°C pendant 4 heures. Le mélange réactionnel est filtré, évaporé à sec, le résidu est mis en suspension dans 100 cm³ d'eau, acidifié par 3,5 cm³ d'une solution aqueuse d'acide chlorhydrique 1N, on additionne 10 cm³ d'éthanol, on chauffe à 100°C et on filtre à chaud. Le résidu est lavé par 3 fois 25 cm³ d'eau, puis par 10 cm³ d'éthanol. On obtient 0,98 g d'acide 9-[3-(3,4-diméthyl-phényl)-pipérazin-1-yl]-8-fluoro-4-méthyl-1-oxo-1,4-dihydro-benzo[f][1,7]naphthyridine-2-carboxylique sous forme d'un solide jaune fondant à 325°C.

Le 9-[3-(3,4-diméthyl-phényl)-pipérazin-1-yl]-8-fluoro-4-méthyl-1-oxo-1,4-dihydrobenzo[f][1,7]naphtyridine-2-carboxylate d'éthyle peut être obtenu de la façon suivante :
Une suspension agitée de 2,5 g de 8,9-difluoro-4-méthyl-1-oxo-1,4-dihydrobenzo[f] [1,7] naphtyridine-2-carboxylate d'éthyle et de 2,6 g de 2-(3,4-diméthylphényl)-pipérazine dans 45 cm³ de diméthylsulfoxyde est chauffée à une température voisine de 100°C pendant environ 100 heures. Le mélange réactionnel est traité avec 400 cm³ d'eau, filtré. Le résidu obtenu est chromatographié sur colonne de gel de silice Merck (granulométrie 0,04-0,06 mm, diamètre 5,5 cm, hauteur 45 cm), en éluant sous une pression de 120 kPa d'argon par un mélange de dichlorométhane et d'éthanol (mélange 92/8 en volumes) et en recueillant des fractions de 50 cm³. Les fractions 37 à 80 sont réunies, puis concentrées à sec sous pression réduite (2,7 kPa). On obtient 1,61 g de 9-[3-(3,4-diméthyl-phényl)-pipérazin-1-yl]-8-fluoro-4-méthyl-1-oxo-1,4-dihydro-benzo[f][1,7]naphtyridine-2-carboxylate d'éthyle sous forme d'un solide jaune fondant à 290°C.

Le 8,9-difluoro-4-méthyl-1-oxo-1,4-dihydrobenzo[f][1,7]naphtyridine-2-carboxylate d'éthyle peut être obtenu comme décrit dans l'exemple 2.

### Exemple 15

L'acide-9-fluoro-8-[4-(4-fluoro-phényl)-pipérazin-1-yl]-4-méthyl-1-oxo-1,4-dihydrobenzo[f][1,7]naphtyridine-2-carboxylique peut être préparé de la façon suivante :
Une suspension de 0,70 g de 9-fluoro-8-[4-(4-fluoro-phényl)-pipérazin-1-yl]-4-méthyl-1-oxo-1,4-dihydro-benzo[f][1,7]naphtyridine-2-carboxylate d'éthyle dans un mélange de 250 cm³ d'éthanol à 40% et 4 cm³ d'une solution aqueuse de potasse 1N est chauffée sous agitation pendant 8 heures à une température de 80°C. Après refroidissement à une température de 60°C, on élimine le léger insoluble par filtration. La solution est neutralisée par 4,1 cm³ d'une solution aqueuse d'acide chlorhydrique 1N; l'insoluble formé est essoré, lavé par 3 fois 20 cm³ d'eau et 2 fois 20 cm³ d'éthanol et séché sous vide à 100°C. On obtient 0,56 g d'acide 9-fluoro-8-[4-(4-fluoro-phényl)-pipérazine- 1-yl]-4-méthyl-1-oxo-1,4-dihydrobenzo[f][1,7]naphtyridine-2-carboxylique sous forme de cristaux jaunes fondant à 346°C.

Le 9-fluoro-8-[4-(4-fluoro-phényl)-pipérazine-1-yl]-4-méthyl-1-oxo-1,4-dihydrobenzo[f][1,7]naphtyridine-2-carboxylate d'éthyle peut être obtenu de la façon suivante:
Une suspension de 1,5 g de 8,9-difluoro-4-méthyl-1-oxo-1,4-dihydrobenzo[f][1,7]naphtyridine-2-carboxylate d'éthyle et de 1,7 g de 1-(4-fluorophényl)pipérazine dans 30 cm³ de diméthylsulfoxyde est chauffée sous agitation à une température de 90°C pendant 4 jours. Après refroidissement à 20°C le mélange est versé dans 150 cm³ d'eau glacée. On extrait par 3 fois 150 cm³ de dichlorométhane; les extraits organiques sont lavés par 3 fois 100 cm³ d'eau et séchés sur sulfate de sodium. Après filtration et concentration sous pression réduite (5,2 kPa) le produit obtenu est purifié par chromatographie sur gel de silice (0,06-0,20 mm). On élue en recueillant des fractions de 200 cm³ par 7,8 litres d'un mélange de dichlorométhane-éthanol (97-3) en volume. Les fractions comprises entre 5,6 et 7,8 litres sont concentrées sous pression réduite (5,2 kPa). On obtient 0,77 g de 9-fluoro-8-[4-(4-fluoro-phényl)-pipérazine-1-yl]-4-méthyl-1-oxo-1,4-dihydrobenzo[f][1,7]-naphtyridine-2-carboxylate d'éthyle sous forme de cristaux jaunes fondant à 300°C.

Le 8,9-difluoro-4-méthyl-1-oxo-1,4-dihydrobenzo[f][1,7]naphtyridine-2-carboxylate d'éthyle peut être obtenu comme décrit dans l'exemple 2.

### Exemple 16

L'acide-9-fluoro-4-méthyl-1-oxo-8-pyrrolidinyl-1,4-dihydrobenzo[f][1,7]naphtyridine-2-carboxylique peut être préparé de la façon suivante :
Une suspension de 0,8 g de 9-fluoro-4-méthyl-1-oxo-8-pyrrolidinyl-1,4-dihydrobenzo[f][1,7]naphtyridine-2-carboxylate d'éthyle dans un mélange de 60 cm³ d'éthanol aqueux à 50% et 2,8 cm³ d'une solution aqueuse de potasse 1N est chauffée sous agitation 2 heures à une température de 70°C. Après concentration sous pression réduite (5,2 kPa) à 60°C le résidu est dissous dans 100 cm³ d'eau; on élimine le léger insoluble par filtration. La solution obtenue est neutralisée par 2,8 cm³ d'une solution aqueuse d'acide chlorhydrique 1N. L'insoluble formé est essoré , lavé par 3 fois 20 cm³ d'eau, 2 fois 15 cm³ d'éthanol et séché sous vide à 100°C. On obtient 0,65 g d'acide 9-fluoro-4-méthyl-1-oxo-8-pyrrolidinyl-1,4-dihydrobenzo[f][1,7]naphtyridine-2-carboxylique sous forme de cristaux jaunes fondant à 365°C.

Le 9-fluoro-4-méthyl-1-oxo-8-pyrrolidinyl-1,4-dihydro-benzo[f][1,7]naphtyridine-2-carboxylate d'éthyle peut être obtenu de la façon suivante :
Une suspension de 2 g de 8,9-difluoro-4-méthyl-1-oxo-1,4-dihydrobenzo[f][1,7]naphtyridine-2-carboxylate d'éthyle et de 1,1 g de pyrrolidine dans 40 cm³ de diméthylformamide est chauffée sous agitation à une température de 70°C pendant 8 heures. Après refroidissement à 20°C ,le mélange est versé dans 150 cm³ d'eau glacée. L'insoluble est essoré, lavé par 3 fois 30 cm³ d'eau, et dissout dans 300 cm³ de dichlorométhane; on décante l'eau résiduelle. Les extraits organiques sont séchés sur sulfate de sodium; après filtration et concentration sous pression réduite (5,2 kPa) le produit obtenu est purifié par chromatographie sur 180 g de gel de silice (0,06-0,20 mm). On élue en recueillant des fractions de 150 cm³ par 3,6 litres d'un mélange de dichlorométhane-éthanol (97-3) en volume et par 6 litres d'un mélange de dichlorométhane-éthanol (94-6) en volume. Les fractions obtenues de ce dernier mélange sont concentrées à sec sous pression réduite. On obtient 0,85 g de 9-fluoro-4-méthyl-1-oxo-8-pyrrolidinyl-1,4-dihydro-benzo[f][1,7]naphtyridine-2-carboxylate d'éthyle sous forme d'un produit jaune fondant à 270°C.

Le 8,9-difluoro-4-méthyl-1-oxo-1,4-dihydrobenzo[f][1,7]naphtyridine-2-carboxylate d'éthyle peut être obtenu comme décrit dans l'exemple 2.

### Exemple 17 :

De façon tout à fait analogue aux méthodes exposées dans les exemples ci-avant, on peut aussi préparer les dérivés suivants, qui sont tout particulièrement intéressants :
l'acide 8-chloro-7-fluoro-9-[4-(3-fluoro-4-méthylphényl)-pipérazin-1-yl]-4-éthyl-1-oxo-1,4-dihydrobenzo[f][1,7]naphtyridine-2-carboxylique,
l'acide-8-fluoro-4-méthyl-1-oxo-9-[4-(3-fluoro-4-méthylphényl)pipérazin-1-yl]-1,4-dihydro-benzo[f][1,7]naphtyridine-2-carboxylique,
l'acide-8-fluoro-4-éthyl-1-oxo-9-[4-(3-fluoro-4-méthylphényl)pipérazin-1-yl]-1,4-dihydro-benzo[f][1,7]naphtyridine-2-carboxylique;
l'acide 8-fluoro-4-méthyl-1-oxo-7-[4-(3-fluoro-4-méthylphényl)pipérazin-1-yl]-1,4-dihydrobenzo[f][1,7]naphtyridine-2-carboxylique,
l'acide 8-fluoro-4-éthyl-1-oxo-7-[4-(3-fluoro-4-méthylphényl)pipérazin-1-yl]-1,4-dihydrobenzo[f][1,7]naphtyridine-2-carboxylique,
l'acide 8-[4-(3-fluoro-4-méthylphényl)pipérazin-1-yl]-9-fluoro-4-méthyl-1-oxo-1,4-dihydrobenzo[f][ 1,7]naphtyridine-2-carboxylique,
l'acide 8-[4-(3-fluoro-4-méthylphényl)pipérazin-1-yl]-9-fluoro-4-éthyl-1-oxo-1,4-dihydrobenzo[f][1,7]naphtyridine-2-carboxylique,
l'acide 7-fluoro-8-[4-(3-fluoro-4-méthylphényl)pipérazin-1-yl]-4-méthyl-1-oxo-1,4-dihydrobenzo[f][1,7] naphtyridine-2-carboxylique,
l'acide 7-fluoro-8-[4-(3-fluoro-4-méthylphényl)pipérazin-1-yl]-4-éthyl-1-oxo-1,4-dihydrobenzo[f][1,7] naphtyridine-2-carboxylique,
l'acide 8-chloro-7-fluoro-9-(1,3,3-triméthyl-6-azabicyclo[3.2.1]oct-6-yl)-4-méthyl-1-oxo-1,4-dihydrobenzo[f][1,7]naphtyridine-2-carboxylique,
l'acide 8-chloro-7-fluoro-9-(1,3,3-triméthyl-6-azabicyclo[3.2.1 ]oct-6-yl)-4-éthyl-1-oxo-1,4-dihydrobenzo[f][1,7]naphtyridine-2-carboxylique,
l'acide-8-fluoro-4-éthyl-1-oxo-9-(1,3,3-triméthyl-6-azabicyclo[3.2.1]oct-6-yl)-1,4-dihydro-benzo[f][1,7]naphtyridine-2-carboxylique,
l'acide 8-fluoro-4-méthyl-1-oxo-7-( 1,3,3-triméthyl-6-azabicyclo[3.2.1]oct-6-yl)-1,4-dihydrobenzo[f][1,7]naphtyridine-2-carboxylique,
l'acide 8-fluoro-4-éthyl-1-oxo-7-(1,3,3-triméthyl-6-azabicyclo[3.2.1]oct-6-yl)-1,4-dihydrobenzo[f][1,7]naphtyridine-2-carboxylique,
l'acide 8-(1,3,3-triméthyl-6-azabicyclo[3.2.1]oct-6-yl)-9-fluoro-4-méthyl-1-oxo-1,4-dihydrobenzo[f][1,7]naphtyridine-2-carboxylique,
l'acide 8-(1,3,3-triméthyl-6-azabicyclo[3.2.1]oct-6-yl)-9-fluoro-4-éthyl-1-oxo-1,4-dihydrobenzo[f] [1,7]naphtyridine-2-carboxylique,
l'acide 7-fluoro-8-(1,3,3-triméthyl-6-azabicyclo[3.2.1]oct-6-yl)-4-méthyl-1-oxo-1,4-dihydrobenzo[f][1,7] naphtyridine-2-carboxylique,
l'acide 7-fluoro-8-(1,3,3-triméthyl-6-azabicyclo[3.2.1]oct-6-yl)-4-éthyl-1-oxo-1,4-dihydrobenzo[f][1,7] naphtyridine-2-carboxylique,
l'acide 8-chloro-7-fluoro-9-[4-(3-chloro-4-fluoro-phényl)pipérazin-1-yl]-4-éthyl-1-oxo-1,4-dihydrobenzo[f][1,7]naphtyridine-2-carboxylique,
l'acide 8-fluoro-4-méthyl-1-oxo-9-[4-(3-chloro-4-fluoro-phényl)pipérazin-1-yl]-1,4-dihydro-benzo[f][1,7]naphtyridine-2-carboxylique,
l'acide 8-fluoro-4-éthyl-1-oxo-9-[4-(3-chloro-4-fluoro-phényl)pipérazin-1-yl]-1,4-dihydrobenzo[f][1,7]naphtyridine-2-carboxylique,
l'acide 8-fluoro-4-méthyl-1-oxo-7-[4-(3 -chloro-4-fluoro-phényl)pipérazin-1-yl]-1,4-dihydrobenzo[f][1,7]naphtyridine-2-carboxylique,
l'acide 8-fluoro-4-éthyl-1-oxo-7-[4-(3-chloro-4-fluoro-phényl)pipérazin-1-yl]-1,4-dihydrobenzo[f][1,7]naphtyridine-2-carboxylique,
l'acide 8-[4-(3-chloro-4-fluoro-phényl)pipérazin-1-yl]-9-fluoro-4-méthyl-1-oxo-1,4-dihydrobenzo[f][ 1,7]naphtyridine-2-carboxylique,
l'acide 8-[4-(3-chloro-4-fluoro-phényl)pipérazin-1-yl]-9-fluoro-4-éthyl-1-oxo-1,4-dihydrobenzo[f][1,7]naphtyridine-2-carboxylique,
l'acide 7-fluoro-8-[4-(3-chloro-4-fluoro-phényl)pipérazin-1-yl]-4-méthyl-1-oxo-1,4-dihydrobenzo[f][1,7]naphtyridine-2-carboxylique,
l'acide 7-fluoro-8-[4-(3 -chloro-4-fluoro-phényl)pipérazin-1-yl]-4-éthyl-1-oxo-1,4-dihydrobenzo[f][1,7] naphtyridine-2-carboxylique,
l'acide 8-chloro-7-fluoro-9-[4-(3-trifluorométhyl-phényl)-pipérazin-1-yl]-4-méthyl-1-oxo-1,4-dihydrobenzo[f][1,7]naphtyridine-2-carboxylique,
l'acide 8-chloro-7-fluoro-9-[4-(3-trifluorométhyl-phényl)-pipérazin-1-yl]-4-éthyl-1-oxo-1,4-dihydrobenzo[f][1,7]naphtyridine-2-carboxylique,
l'acide 8-fluoro-4-méthyl-1-oxo-9-[4-(3-trifluorométhyl-phényl)-pipérazin-1-yl]-1,4-dihydrobenzo[f][1,7]naphtyridine-2-carboxylique,
l'acide 8-fluoro-4-éthyl-1-oxo-9-[4-(3-trifluorométhyl-phényl)-pipérazin-1-yl]-1,4-dihydrobenzo[f][1,7]naphtyridine-2-carboxylique,
l'acide 8-fluoro-4-éthyl-1-oxo-7-[4-(3-trifluorométhyl-phényl)-pipérazin-1-yl]-1,4-dihydrobenzo[f][1,7]naphtyridine-2-carboxylique,
l'acide 8-[4-(3-trifluorométhyl-phényl)-pipérazin-1-yl]-9-fluoro-4-méthyl-1-oxo-1,4-dihydrobenzo[f][ 1,7]naphtyridine-2-carboxylique,
l'acide 8-[4-(3-trifluorométhyl-phényl)-pipérazin-1-yl]-9-fluoro-4-éthyl-1-oxo-1,4-dihydrobenzo[f][1,7]naphtyridine-2-carboxylique,
l'acide 7-fluoro-8-[4-(3-trifluorométhyl-phényl)-pipérazin-1-yl]-4-méthyl-1-oxo-1,4-dihydrobenzo[f][1,7] naphtyridine-2-carboxylique,
l'acide 7-fluoro-8-[4-(3-trifluorométhyl-phényl)-pipérazin-1-yl]-4-éthyl-1-oxo-1,4-dihydrobenzo[f][1,7] naphtyridine-2-carboxylique,
l'acide 8-chloro-7-fluoro-9-[3,3-diméthylpipéridino]-4-méthyl-1-oxo-1,4-dihydrobenzo[f][1,7]naphtyridine-2-carboxylique,
l'acide 8-chloro-7-fluoro-9-[3,3-diméthylpipéridino]-4-éthyl-1-oxo-1,4-dihydrobenzo [f] [1,7] naphtyridine-2-carboxylique,
l'acide-8-fluoro-4-éthyl-1-oxo-9-[3,3-diméthylpipéridino]-1,4-dihydrobenzo[f] [1,7] naphtyridine-2-carboxylique,
l'acide 8-fluoro-4-méthyl-1-oxo-7-[3,3-diméthylpipéridino]-1,4-dihydrobenzo[f] [1,7] naphtyridine-2-carboxylique,
l'acide 8-fluoro-4-éthyl-1-oxo-7-[3,3-diméthylpipéridino]-1,4-dihydrobenzo[f] [1,7] naphtyridine-2-carboxylique,
l'acide 8-[3,3-diméthylpipéridino]-9-fluoro-4-méthyl-1-oxo-1,4-dihydrobenzo[f] [1,7] naphtyridine-2-carboxylique,
l'acide 8-[3,3-diméthylpipéridino]-9-fluoro-4-éthyl-1-oxo-1,4-dihydrobenzo[f] [1,7] naphtyridine-2-carboxylique,
l'acide 7-fluoro-8-[3,3-diméthylpipéridino]-4-méthyl-1-oxo-1,4-dihydrobenzo[f] [1,7] naphtyridine-2-carboxylique,
l'acide 7-fluoro-8-[3,3-diméthylpipéridino]-4-éthyl-1-oxo-1,4-dihydrobenzo[f] [1,7] naphtyridine-2-carboxylique,
l'acide 8-chloro-7-fluoro-9-[4-hydroxy-4-(3-trifluorométhyl-phényl)-pipéridin-1-yl]-4-méthyl-1-oxo-1,4-dihydrobenzo[f][1,7]naphtyridine-2-carboxylique,
l'acide 8-chloro-7-fluoro-9-[4-hydroxy-4-(3-trifluorométhyl-phényl)-pipéridin-1-yl]-4-éthyl-1-oxo-1,4-dihydrobenzo[f][1,7]naphtyridine-2-carboxylique,
l'acide 8-fluoro-4-méthyl-1-oxo-9-[4-hydroxy-4-(3-trifluorométhyl-phényl)-pipéridin-1-yl]-1,4-dihydro-benzo[f][1,7]naphtyridine-2-carboxylique,
l'acide 8-fluoro-4-éthyl-1-oxo-9-[4-hydroxy-4-(3-trifluorométhyl-phényl)-pipéridin-1-yl]-1,4-dihydro-benzo[f][1,7]naphtyridine-2-carboxylique,
l'acide 8-fluoro-4-éthyl-1-oxo-7-[4-hydroxy-4-(3-trifluorométhyl-phényl)-pipéridin-1-yl]-1,4-dihydrobenzo[f][1,7]naphtyridine-2-carboxylique,
l'acide 8-[4-hydroxy-4-(3-trifluorométhyl-phényl)-pipéridin-1-yl]-9-fluoro-4-méthyl-1-oxo-1,4-dihydrobenzo[f][1,7]naphtyridine-2-carboxylique,
l'acide 8-[4-hydroxy-4-(3-trifluorométhyl-phényl)-pipéridin-1-yl]-9-fluoro-4-éthyl-1-oxo-1,4-dihydrobenzo[f][1,7]naphtyridine-2-carboxylique,
l'acide 7-fluoro-8-[4-hydroxy-4-(3-trifluorométhyl-phényl)-pipéridin-1-yl]-4-méthyl-1-oxo-1,4-dihydrobenzo[f][1,7] naphtyridine-2-carboxylique,
l'acide 7-fluoro-8-[4-hydroxy-4-(3-trifluorométhyl-phényl)-pipéridin-1-yl]-4-éthyl-1-oxo-1,4-dihydrobenzo[f][1,7] naphtyridine-2-carboxylique,
l'acide 8-chloro-7-fluoro-9-[2-anilinométhyl-(2S)-pyrrolidino]-4-méthyl-1-oxo-1,4-dihydrobenzo[f] [1,7]naphtyridine-2-carboxylique,
l'acide 8-chloro-7-fluoro-9-[2-anilinométhyl-(2S)-pyrrolidino]-4-éthyl-1-oxo-1,4-dihydrobenzo[f][1,7]naphtyridine-2-carboxylique,
l'acide-8-fluoro-4-éthyl-1-oxo-9-[2-anilinométhyl-(2S)-pyrrolidino]-1,4-dihydrobenzo[f][1,7]naphtyridine-2-carboxylique,
l'acide 8-fluoro-4-méthyl-1-oxo-7-[2-anilinométhyl-(2S)-pyrrolidino]-1,4-dihydrobenzo[f][1,7]naphtyridine-2-carboxylique,
l'acide 8-fluoro-4-éthyl-1-oxo-7-[2-anilinométhyl-(2S)-pyrrolidino]-1,4-dihydrobenzo[f][1,7]naphtyridine-2-carboxylique,
l'acide 8-[2-anilinométhyl-(2S)-pyrrolidino]-9-fluoro-4-méthyl-1-oxo-1,4-dihydrobenzo[f][1,7]naphtyridine-2-carboxylique,
l'acide 8-[2-anilinométhyl-(2S)-pyrrolidino]-9-fluoro-4-éthyl-1-oxo-1,4-dihydrobenzo[f][1,7]naphtyridine-2-carboxylique,
l'acide 7-fluoro-8-[2-anilinométhyl-(2S)-pyrrolidino]-4-méthyl-1-oxo-1,4-dihydrobenzo[f][1,7] naphtyridine-2-carboxylique,
l'acide 7-fluoro-8-[2-anilinométhyl-(2S)-pyrrolidino]-4-éthyl-1-oxo-1,4-dihydrobenzo[f][1,7]naphtyridine-2-carboxylique,
l'acide 8-chloro-7-fluoro-9-[3-(4-benzyloxy-3-méthoxy-phényl)-pipérazin-1-yl]-4-méthyl-1-oxo-1,4-dihydrobenzo[f][1,7]naphtyridine-2-carboxylique,
l'acide 8-chloro-7-fluoro-9-[3-(4-benzyloxy-3-méthoxy-phényl)-pipérazin-1-yl]-4-éthyl-1-oxo-1,4-dihydrobenzo[f][1,7]naphtyridine-2-carboxylique,
l'acide 8-fluoro-4-éthyl-1-oxo-9-[3-(4-benzyloxy-3-méthoxy-phényl)-pipérazin-1-yl]-1,4-dihydro-benzo[f][1,7]naphtyridine-2-carboxylique,
l'acide 8-fluoro-4-méthyl-1-oxo-7-[3-(4-benzyloxy-3 -méthoxy-phényl)-pipérazin-1-yl]-1,4-dihydrobenzo[f][1,7]naphtyridine-2-carboxylique,
l'acide 8-fluoro-4-éthyl-1-oxo-7-[3-(4-benzyloxy-3-méthoxy-phényl)-pipérazin-1-yl]-1,4-dihydrobenzo[f][1,7]naphtyridine-2-carboxylique,
l'acide 8-[3-(4-benzyloxy-3-méthoxy-phényl)-pipérazin-1-yl]-9-fluoro-4-méthyl-1-oxo-1,4-dihydrobenzo[f][1,7]naphtyridine-2-carboxylique,
l'acide 8-[3-(4-benzyloxy-3-méthoxy-phényl)-pipérazin-1-yl]-9-fluoro-4-éthyl-1-oxo-1,4-dihydrobenzo[f][1,7]naphtyridine-2-carboxylique,
l'acide 7-fluoro-8-[3-(4-benzyloxy-3-méthoxy-phényl)-pipérazin-1-yl]-4-méthyl-1-oxo-1,4-dihydrobenzo[f][1,7] naphtyridine-2-carboxylique,
l'acide 7-fluoro-8-[3-(4-benzyloxy-3-méthoxy-phényl)-pipérazin-1-yl]-4-éthyl-1-oxo-1,4-dihydrobenzo[f][1,7] naphtyridine-2-carboxylique,
l'acide 8-chloro-7-fluoro-9-[4-(3-méthoxyphényl)-3-méthylpipérazin-1-yl]-4-méthyl-1-oxo-1,4-dihydrobenzo[f][1,7]naphtyridine-2-carboxylique,
l'acide 8-chloro-7-fluoro-9-[4-(3-méthoxyphényl)-3-méthylpipérazin-1-yl]-4-éthyl-1-oxo-1,4-dihydrobenzo[f][1,7]naphtyridine-2-carboxylique,
l'acide 8-fluoro-4-éthyl-1-oxo-9-[4-(3-méthoxyphényl)-3-méthylpipérazin-1-yl]-1,4-dihydro-benzo[f][1,7]naphtyridine-2-carboxylique,
l'acide 8-fluoro-4-méthyl-1-oxo-7-[4-(3-méthoxyphényl)-3-méthylpipérazin-1-yl]-1,4-dihydrobenzo[f][1,7]naphtyridine-2-carboxylique,
l'acide 8-fluoro-4-éthyl-1-oxo-7-[4-(3-méthoxyphényl)-3-méthylpipérazin-1-yl]-1,4-dihydrobenzo[f][1,7]naphtyridine-2-carboxylique,
l'acide 8-[4-(3-méthoxyphényl)-3-méthylpipérazin-1-yl]-9-fluoro-4-méthyl- 1-oxo-1,4-dihydrobenzo[f][1,7]naphtyridine-2-carboxylique,
l'acide 8-[4-(3-méthoxyphényl)-3-méthylpipérazin-1-yl]-9-fluoro-4-éthyl-1-oxo-1,4-dihydrobenzo[f][1,7]naphtyridine-2-carboxylique,
l'acide 7-fluoro-8-[4-(3-méthoxyphényl)-3-méthylpipérazin-1-yl]-4-méthyl-1-oxo-1,4-dihydrobenzo[f][1,7] naphtyridine-2-carboxylique, l'acide 7-fluoro-8-[4-(3-méthoxyphényl)-3-méthylpipérazin-1-yl]-4-éthyl-1-oxo-1,4-dihydrobenzo[f][1,7] naphtyridine-2-carboxylique,
l'acide 8-chloro-7-fluoro-9-[3-(3,4-diméthyl-phényl)-pipérazin-1-yl]-4-méthyl-1-oxo-1,4-dihydrobenzo[f][[1,7]naphtyridine-2-carboxylique,
l'acide 8-chloro-7-fluoro-9-[3-(3,4-diméthyl-phényl)-pipérazin-1-yl]-4-éthyl-1-oxo-1,4-dihydrobenzo[f][1,7]naphtyridine-2-carboxylique,
l'acide 8-fluoro-4-éthyl-1-oxo-9-[3-(3,4-diméthyl-phényl)-pipérazin-1-yl]-1,4-dihydrobenzo[f][1,7]naphtyridine-2-carboxylique,
l'acide 8-fluoro-4-méthyl-1-oxo-7-[3-(3,4-diméthyl-phényl)-pipérazin-1-yl]-1,4-dihydrobenzo[f][1,7]naphtyridine-2-carboxylique,
l'acide 8-fluoro-4-éthyl-1-oxo-7-[3-(3,4-diméthyl-phényl)-pipérazin-1-yl]-1,4-dihydrobenzo[f][1,7]naphtyridine-2-carboxylique,
l'acide 8-[3-(3,4-diméthyl-phényl)-pipérazin-1-yl]-9-fluoro-4-méthyl-1-oxo-1,4-dihydrobenzo[f] [1,7]naphtyridine-2-carboxylique,
l'acide 8-[3-(3,4-diméthyl-phényl)-pipérazin-1-yl]-9-fluoro-4-éthyl-1-oxo- 1,4-dihydrobenzo[f][1,7]naphtyridine-2-carboxylique,
l'acide 7-fluoro-8-[3-(3,4-diméthyl-phényl)-pipérazin-1-yl]-4-méthyl-1-oxo-1,4-dihydrobenzo[f][1,7] naphtyridine-2-carboxylique,
l'acide 7-fluoro-8-[3-(3,4-diméthyl-phényl)-pipérazin-1-yl]-4-éthyl-1-oxo-1,4-dihydrobenzo[f][1,7] naphtyridine-2-carboxylique,
l'acide 8-chloro-7-fluoro-9-[4-(4-fluoro-phényl)-pipérazin-1-yl]-4-méthyl-1-oxo-1,4-dihydrobenzo[f][1,7]naphtyridine-2-carboxylique,
l'acide 8-chloro-7-fluoro-9-[4-(4-fluoro-phényl)-pipérazin-1-yl]-4-éthyl-1-oxo-1,4-dihydrobenzo[f][1,7]naphtyridine-2-carboxylique,
l'acide 8-fluoro-4-méthyl-1-oxo-9-[4-(4-fluoro-phényl)-pipérazin-1-yl]-1,4-dihydrobenzo[f][1,7]naphtyridine-2-carboxylique,
l'acide 8-fluoro-4-éthyl-1-oxo-9-[4-(4-fluoro-phényl)-pipérazin-1-yl]-1,4-dihydrobenzo[f][1,7]naphtyridine-2-carboxylique,
l'acide 8-fluoro-4-méthyl-1-oxo-7-[4-(4-fluoro-phényl)-pipérazin-1-yl]-1,4-dihydrobenzo[f][1,7]naphtyridine-2-carboxylique,
l'acide 8-fluoro-4-éthyl-1-oxo-7-[4-(4-fluoro-phényl)-pipérazin-1-yl]-1,4-dihydrobenzo[f][1,7]naphtyridine-2-carboxylique,
l'acide 8-[4-(4-fluoro-phényl)-pipérazin-1-yl]-9-fluoro-4-éthyl-1-oxo-1,4-dihydrobenzo[f][1,7]naphtyridine-2-carboxylique,
l'acide 7-fluoro-8-[4-(4-fluoro-phényl)-pipérazin-1-yl]-4-méthyl-1-oxo-1,4-dihydrobenzo[f][1,7] naphtyridine-2-carboxylique,
l'acide 7-fluoro-8-[4-(4-fluoro-phényl)-pipérazin-1-yl]-4-éthyl-1-oxo-1,4-dihydrobenzo[f] [1,7] naphtyridine-2-carboxylique,
l'acide 8-chloro-7-fluoro-9-(pyrrolidinyl)-4-méthyl-1-oxo-1,4-dihydrobenzo[f] [1,7] naphtyridine-2-carboxylique,
l'acide 8-chloro-7-fluoro-9-(pyrrolidinyl)-4-éthyl-1 -oxo-1,4-dihydrobenzo[f] [1,7] naphtyridine-2-carboxylique,
l'acide 8-fluoro-4-méthyl-1-oxo-9-(pyrrolidinyl)-1,4-dihydro-benzo[f] [1,7] naphtyridine-2-carboxylique,
l'acide 8-fluoro-4-éthyl-1-oxo-9-(pyrrolidinyl)-1,4-dihydro-benzo[f] [1,7] naphtyridine-2-carboxylique,
l'acide 8-fluoro-4-méthyl-1-oxo-7-(pyrrolidinyl)-1,4-dihydrobenzo[f] [1,7] naphtyridine-2-carboxylique,
l'acide 8-fluoro-4-éthyl-1-oxo-7-(pyrrolidinyl)-1,4-dihydrobenzo[f] [1,7] naphtyridine-2-carboxylique,
l'acide 8-(pyrrolidinyl)-9-fluoro-4-éthyl-1-oxo-1,4-dihydrobenzo[f] [1,7] naphtyridine-2-carboxylique,
l'acide 7-fluoro-8-(pyrrolidinyl)-4-méthyl-1-oxo-1,4-dihydrobenzo[f] [1,7] naphtyridine-2-carboxylique,
l'acide 7-fluoro-8-(pyrrolidinyl)-4-éthyl-1-oxo-1,4-dihydrobenzo[f] [1,7] naphtyridine-2-carboxylique,
l'acide 8-chloro-7-fluoro-9-(3,3-diméthyl pyrrolidinyl)-4-méthyl-1-oxo-1,4-dihydrobenzo[f][1,7]naphtyridine-2-carboxylique,
l'acide 8-chloro-7-fluoro-9-(3,3-diméthyl pyrrolidinyl)-4-éthyl-1-oxo-1,4-dihydrobenzo[f][1,7]naphtyridine-2-carboxylique,
l'acide 8-fluoro-4-méthyl-1-oxo-9-(3,3-diméthyl pyrrolidinyl)-1,4-dihydrobenzo[f] [1,7] naphtyridine-2-carboxylique,
l'acide 8-fluoro-4-éthyl-1-oxo-9-(3,3-diméthyl pyrrolidinyl)-1,4-dihydrobenzo[f] [1,7] naphtyridine-2-carboxylique,
l'acide 8-fluoro-4-méthyl-1-oxo-7-(3,3-diméthyl pyrrolidinyl)-1,4-dihydrobenzo[f] [1,7] naphtyridine-2-carboxylique,
l'acide 8-fluoro-4-éthyl-1-oxo-7-(3,3-diméthyl pyrrolidinyl)-1,4-dihydrobenzo[f] [1,7] naphtyridine-2-carboxylique,
l'acide 8-(3,3-diméthyl pyrrolidinyl)-9-fluoro-4-méthyl-1-oxo-1,4-dihydrobenzo[f] [1,7]naphtyridine-2-carboxylique,
l'acide 8-(3,3-diméthyl pyrrolidinyl)-9-fluoro-4-éthyl-1-oxo-1,4-dihydrobenzo[f] [1,7] naphtyridine-2-carboxylique,
l'acide 7-fluoro-8-(3,3-diméthyl pyrrolidinyl)-4-méthyl-1-oxo-1,4-dihydrobenzo[f] [1,7] naphtyridine-2-carboxylique,
l'acide 7-fluoro-8-(3,3-diméthyl pyrrolidinyl)-4-éthyl-1-oxo-1,4-dihydrobenzo[f] [1,7] naphtyridine-2-carboxylique,
l'acide 8-chloro-7-fluoro-9-(3-hydroxy pyrrolidinyl)-4-méthyl-1-oxo-1,4-dihydrobenzo[f][1,7]naphtyridine-2-carboxylique,
l'acide 8-chloro-7-fluoro-9-(3-hydroxy pyrrolidinyl)-4-éthyl-1-oxo-1,4-dihydrobenzo[f] [1,7]naphtyridine-2-carboxylique,
l'acide 8-fluoro-4-méthyl-1-oxo-9-(3-hydroxy pyrrolidinyl)-1,4-dihydrobenzo[f] [1,7] naphtyridine-2-carboxylique,
l'acide 8-fluoro-4-éthyl-1-oxo-9-(3-hydroxy pyrrolidinyl)-1,4-dihydrobenzo[fl [1,7] naphtyridine-2-carboxylique,
l'acide 8-fluoro-4-méthyl-1-oxo-7-(3-hydroxy pyrrolidinyl)-1,4-dihydrobenzo[f] [1,7] naphtyridine-2-carboxylique,
l'acide 8-fluoro-4-éthyl-1-oxo-7-(3-hydroxy pyrrolidinyl)-1,4-dihydrobenzo[f] [1,7] naphtyridine-2-carboxylique,
l'acide 8-(3-hydroxy pyrrolidinyl)-9-fluoro-4-méthyl-1-oxo-1,4-dihydrobenzo[fl [1,7] naphtyridine-2-carboxylique,
l'acide 8-(3-hydroxy pyrrolidinyl)-9-fluoro-4-éthyl-1-oxo-1,4-dihydrobenzo[f] [1,7] naphtyridine-2-carboxylique,
l'acide 7-fluoro-8-(3-hydroxy pyrrolidinyl)-4-méthyl-1-oxo-1,4-dihydrobenzo[f] [1,7] naphtyridine-2-carboxylique,
l'acide 7-fluoro-8-(3-hydroxy pyrrolidinyl)-4-éthyl-1-oxo-1,4-dihydrobenzo[f] [1,7] naphtyridine-2-carboxylique,
l'acide 8-chloro-7-fluoro-9-(pipéridinyl)-4-méthyl-1-oxo-1,4-dihydrobenzo[f] [1,7] naphtyridine-2-carboxylique,
l'acide 8-chloro-7-fluoro-9-(pipéridinyl)-4-éthyl-1-oxo-1,4-dihydrobenzo[f] [1,7] naphtyridine-2-carboxylique,
l'acide 8-fluoro-4-méthyl-1-oxo-9-(pipéridinyl)-1,4-dihydrobenzo [f] [1,7] naphtyridine-2-carboxylique,
l'acide 8-fluoro-4-éthyl-1-oxo-9-(pipéridinyl)-1,4-dihydrobenzo[f][1,7] naphtyridine-2-carboxylique,
l'acide 8-fluoro-4-méthyl-1-oxo-7-(pipéridinyl)-1,4-dihydrobenzo[f] [1,7] naphtyridine-2-carboxylique,
l'acide 8-fluoro-4-éthyl-1-oxo-7-(pipéridinyl)-1,4-dihydrobenzo[f] [1,7] naphtyridine-2-carboxylique,
l'acide 8-(pipéridinyl)-9-fluoro-4-méthyl-1-oxo-1,4-dihydrobenzo[f] [1,7] naphtyridine-2-carboxylique,
l'acide 8-(pipéridinyl)-9-fluoro-4-éthyl-1-oxo-1,4-dihydrobenzo[f] [1,7] naphtyridine-2-carboxylique,
l'acide 7-fluoro-8-(pipéridinyl)-4-méthyl-1-oxo-1,4-dihydrobenzo[f] [1,7] naphtyridine-2-carboxylique,
l'acide 7-fluoro-8-(pipéridinyl)-4-éthyl-1-oxo-1,4-dihydrobenzo[f] [1,7] naphtyridine-2-carboxylique,
l'acide 8-chloro-7-fluoro-9-(3-hydroxy pipéridinyl)-4-méthyl-1-oxo-1,4-dihydrobenzo[f] [1,7]naphtyridine-2-carboxylique,
l'acide 8-chloro-7-fluoro-9-(3-hydroxy pipéridinyl)-4-éthyl-1-oxo-1,4-dihydrobenzo[f][1,7]naphtyridine-2-carboxylique,
l'acide 8-fluoro-4-méthyl-1-oxo-9-(3-hydroxy pipéridinyl)-1,4-dihydrobenzo[f] [1,7] naphtyridine-2-carboxylique,
l'acide 8-fluoro-4-éthyl-1-oxo-9-(3-hydroxy pipéridinyl)-1,4-dihydrobenzo[f] [1,7] naphtyridine-2-carboxylique,
l'acide 8-fluoro-4-méthyl-1-oxo-7-(3-hydroxy pipéridinyl)-1,4-dihydrobenzo[f] [1,7] naphtyridine-2-carboxylique,
l'acide 8-fluoro-4-éthyl-1-oxo-7-(3-hydroxy pipéridinyl)-1,4-dihydrobenzo[f] [1,7] naphtyridine-2-carboxylique,
l'acide 8-(3-hydroxy pipéridinyl)-9-fluoro-4-méthyl-1-oxo-1,4-dihydrobenzo[f] [1,7] naphtyridine-2-carboxylique,
l'acide 8-(3-hydroxy pipéridinyl)-9-fluoro-4-éthyl-1-oxo-1,4-dihydrobenzo[f] [1,7] naphtyridine-2-carboxylique,
l'acide 7-fluoro-8-(3-hydroxy pipéridinyl)-4-méthyl-1-oxo-1,4-dihydrobenzo[f][1,7] naphtyridine-2-carboxylique,
l'acide 7-fluoro-8-(3-hydroxy pipéridinyl)-4-éthyl-1-oxo-1,4-dihydrobenzo[f][1,7] naphtyridine-2-carboxylique,
l'acide 8-chloro-7-fluoro-9-(4,4-diméthyl pipéridinyl)-4-méthyl-1-oxo-1,4-dihydrobenzo[f][1,7]naphtyridine-2-carboxylique,
l'acide 8-chloro-7-fluoro-9-(4,4-diméthyl pipéridinyl)-4-éthyl-1-oxo-1,4-dihydrobenzo[f][1,7]naphtyridine-2-carboxylique,
l'acide 8-fluoro-4-méthyl-1-oxo-9-(4,4-diméthyl pipéridinyl)-1,4-dihydrobenzo[f][1,7] naphtyridine-2-carboxylique,
l'acide 8-fluoro-4-éthyl-1-oxo-9-(4,4-diméthyl pipéridinyl)-1,4-dihydrobenzo[f] [1,7] naphtyridine-2-carboxylique,
l'acide 8-fluoro-4-méthyl-1-oxo-7-(4,4-diméthyl pipéridinyl)-1,4-dihydrobenzo[f] [1,7] naphtyridine-2-carboxylique,
l'acide 8-fluoro-4-éthyl-1-oxo-7-(4,4-diméthyl pipéridinyl)-1,4-dihydrobenzo[f] [1,7] naphtyridine-2-carboxylique,
l'acide 8-(4,4-diméthyl pipéridinyl)-9-fluoro-4-méthyl-1-oxo-1,4-dihydrobenzo[f] [1,7] naphtyridine-2-carboxylique,
l'acide 8-(4,4-diméthyl pipéridinyl)-9-fluoro-4-éthyl-1-oxo-1,4-dihydrobenzo[f] [1,7] naphtyridine-2-carboxylique,
l'acide 7-fluoro-8-(4,4-diméthyl pipéridinyl)-4-méthyl-1-oxo-1,4-dihydrobenzo[f] [1,7] naphtyridine-2-carboxylique,
l'acide 7-fluoro-8-(4,4-diméthyl pipéridinyl)-4-éthyl-1-oxo-1,4-dihydrobenzo[f] [1,7] naphtyridine-2-carboxylique,
l'acide 8-chloro-7-fluoro-9-[4-(3,4-difluoro-phényl)pipérazin-1-yl]-4-méthyl-1-oxo-1,4-dihydrobenzo[f][1,7]naphtyridine-2-carboxylique,
l'acide 8-chloro-7-fluoro-9-[4-(3,4-difluoro-phényl)pipérazin-1-yl]-4-éthyl-1-oxo-1,4-dihydrobenzo[f][1,7] naphtyridine-2-carboxylique,
l'acide 8-fluoro-4-méthyl-1-oxo-9-[4-(3,4-difluoro-phényl)pipérazin-1-yl]-1,4-dihydrobenzo[f][1,7] naphtyridine-2-carboxylique,
l'acide 8-fluoro-4-éthyl-1-oxo-9-[4-(3,4-difluoro-phényl)pipérazin-1-yl]-1,4-dihydrobenzo[f][1,7] naphtyridine-2-carboxylique,
l'acide 8-fluoro-4-méthyl-1-oxo-7-[4-(3,4-difluoro-phényl)pipérazin-1-yl]-1,4-dihydrobenzo[f][1,7] naphtyridine-2-carboxylique,
l'acide 8-fluoro-4-éthyl-1-oxo-7-[4-(3,4-difluoro-phényl)pipérazin-1-yl]-1,4-dihydrobenzo[f][1,7] naphtyridine-2-carboxylique,
l'acide 8-[4-(3,4-difluoro-phényl)pipérazin-1-yl]-9-fluoro-4-méthyl-1-oxo- 1,4-dihydrobenzo[f][1,7] naphtyridine-2-carboxylique,
l'acide 8-[4-(3,4-difluoro-phényl)pipérazin-1-yl]-9-fluoro-4-éthyl-1-oxo-1,4-dihydrobenzo[f][1,7] naphtyridine-2-carboxylique,
l'acide 7-fluoro-8-[4-(3,4-difluoro-phényl)pipérazin-1-yl]-4-méthyl-1-oxo-1,4-dihydrobenzo[f][1,7] naphtyridine-2-carboxylique,
l'acide 7-fluoro-8-[4-(3,4-difluoro-phényl)pipérazin-1-yl]-4-éthyl-1-oxo-1,4-dihydrobenzo[f][1,7] naphtyridine-2-carboxylique,
l'acide 8-chloro-7-fluoro-9-[4-hydroxy-4-(4-fluorophényl)-pipéridinyl]-4-méthyl-1-oxo-1,4-dihydrobenzo[f] [1,7]naphtyridine-2-carboxylique,
l'acide 8-chloro-7-fluoro-9-[4-hydroxy-4-(4-fluorophényl)-pipéridinyl]-4-éthyl-1-oxo-1,4-dihydrobenzo[f] [1,7]naphtyridine-2-carboxylique,
l'acide 8-fluoro-4-méthyl-1-oxo-9-[4-hydroxy-4-(4-fluorophényl)-pipéridinyl]-1,4-dihydrobenzo[f] [1,7]naphtyridine-2-carboxylique,
l'acide 8-fluoro-4-éthyl-1-oxo-9-[4-hydroxy-4-(4-fluorophényl)-pipéridinyl]-1,4-dihydrobenzo[f] [1,7]naphtyridine-2-carboxylique,
l'acide 8-fluoro-4-méthyl-1-oxo-7-[4-hydroxy-4-(4-fluorophényl)-pipéridinyl]-1,4-dihydrobenzo[f][1,7]naphtyridine-2-carboxylique,
l'acide 8-fluoro-4-éthyl-1-oxo-7-[4-hydroxy-4-(4-fluorophényl)-pipéridinyl]-1,4-dihydrobenzo[f][1,7]naphtyridine-2-carboxylique,
l'acide 8-[4-hydroxy-4-(4-fluorophényl)-pipéridinyl]-9-fluoro-4-méthyl-1-oxo-1,4-dihydrobenzo[f][1,7]naphtyridine-2-carboxylique,
l'acide 8-[4-hydroxy-4-(4-fluorophényl)-pipéridinyl]-9-fluoro-4-éthyl-1-oxo-1,4-dihydrobenzo[f][1,7]naphtyridine-2-carboxylique,
l'acide 7-fluoro-8-[4-hydroxy-4-(4-fluorophényl)-pipéridinyl]-4-méthyl-1-oxo-1,4-dihydrobenzo[f][1,7]naphtyridine-2-carboxylique,
l'acide 7-fluoro-8-[4-hydroxy-4-(4-fluorophényl)-pipéridinyl]-4-éthyl-1-oxo-1,4-dihydrobenzo[f][1,7] naphtyridine-2-carboxylique,
l'acide 8-chloro-7-fluoro-9-(4-hydroxy 4-méthyl pipéridinyl)-4-méthyl-1-oxo-1,4-dihydrobenzo[f][1,7]naphtyridine-2-carboxylique,
l'acide 8-chloro-7-fluoro-9-(4-hydroxy 4-méthyl pipéridinyl)-4-éthyl-1-oxo-1,4-dihydrobenzo[f][1,7]naphtyridine-2-carboxylique,
l'acide 8-fluoro-4-méthyl-1-oxo-9-(4-hydroxy 4-méthyl pipéridinyl)-1,4-dihydrobenzo[f][1,7]naphtyridine-2-carboxylique,
l'acide 8-fluoro-4-éthyl-1-oxo-9-(4-hydroxy 4-méthyl pipéridinyl)-1,4-dihydrobenzo[f][ 1,7]naphtyridine-2-carboxylique,
l'acide 8-fluoro-4-méthyl-1-oxo-7-(4-hydroxy 4-méthyl pipéridinyl)-1,4-dihydrobenzo[f][1,7]naphtyridine-2-carboxylique,
l'acide 8-fluoro-4-éthyl-1-oxo-7-(4-hydroxy 4-méthyl pipéridinyl)-1,4-dihydrobenzo[f][1,7]naphtyridine-2-carboxylique,
l'acide 8-(4-hydroxy 4-méthyl pipéridinyl)-9-fluoro-4-méthyl-1-oxo-1,4-dihydrobenzo[f] [1,7]naphtyridine-2-carboxylique,
l'acide 8-(4-hydroxy 4-méthyl pipéridinyl)-9-fluoro-4-éthyl-1-oxo-1,4-dihydrobenzo[f][1,7]naphtyridine-2-carboxylique,
l'acide 7-fluoro-8-(4-hydroxy 4-méthyl pipéridinyl)-4-méthyl-1-oxo-1,4-dihydrobenzo[f][1,7] naphtyridine-2-carboxylique,
l'acide 7-fluoro-8-(4-hydroxy 4-méthyl pipéridinyl)-4-éthyl-1-oxo-1,4-dihydrobenzo[f][1,7] naphtyridine-2-carboxylique.

La présente invention concerne également les compositions pharmaceutiques utilisables en médecine humaine ou vétérinaire qui contiennent comme produit actif au moins un produit de formule générale (I) à l'état pur (sous forme libre ou sous forme de sel) ou sous forme d'une association avec un ou plusieurs diluants ou adjuvants compatibles et pharmaceutiquement acceptables. Ces compositions peuvent être utilisées par voie topique.

Les compositions pour administration topique peuvent être par exemple des crèmes, des pommades, des gels, des lotions, des liniments ou des aérosols. Il peut s'agir également de compositions solides pulvérulentes.

Lorsque les compositions sont des crèmes, des pommades ou des gels, ces compositions peuvent être par exemple des pommades hydrophiles contenant par exemple des polyéthylèneglycols et des quantités appropriées d'eau, des pommades hydrophobes contenant par exemple de la vaseline, de la paraffine, de la paraffine liquide, des huiles végétales ou des graisses animales, des glycérides synthétiques, des cires ou des polyalkylsiloxanes liquides, elles peuvent être également des crèmes hydrophiles contenant notamment des agents émulsifiants huile dans eau comme par exemple des savons de sodium ou de triéthanolamine, des alcools gras, des alcools gras sulfatés, des polysorbates en combinaison éventuellement avec des agents émulsifiants eau dans huile, ou des crèmes hydrophobes contenant notamment des agents émulsifiants eau dans huile tels que la graisse de laine, les esters de sorbitannes, les monoglycérides, elles peuvent être aussi des gels hydrophiles à base d'eau, d'alcool, de glycérol ou de propylène glycol gélifiés, ou des gels hydrophobes comprenant de la paraffine liquide additionnée de polyéthylène, des huiles grasses gélifiées par l'oxyde de silicium colloïdal ou des savons d'aluminium ou de zinc.

A titre d'exemple, lorsque les compositions sont des aérosols, pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine ou vétérinaire, les compositions selon l'invention sont particulièrement utiles dans le traitement prophylactique ou curatif des infections cutanéo-muqueuses d'origine bactérienne à Gram-positif, en particulier le traitement curatif des infections cutanées à bactéries Gram positif et/ou le traitement préventif des infections à bactéries Gram positif multirésistantes. Notamment dans le traitement des infections associées à des plaies, des greffes ou des brûlures, dans le traitement des infections liées aux lésions de la peau, ou dans le traitement des impétigos et des furonculoses, ainsi également que pour la prévention de la contamination des voies nasales par les bactéries Gram-positif multirésistantes, et aussi pour la décontamination en vue d'éviter la dissémination de ces germes.

D'une façon générale, le médecin déterminera la posologie qu'il estime la plus appropriée en fonction de l'âge, du degré de l'infection et des autres facteurs propres au sujet à traiter. Généralement, le principe actif est contenu à 1 ou 2 % dans la formulation. Cette formulation est appliquée 2 à 3 fois par jour par voie topique.

L'exemple suivant, donné à titre non limitatif, illustre une composition selon l'invention :

### Exemple de composition :

On prépare selon les techniques habituelles une crème dosée à 2 % d'acide libre, ayant la composition suivante :
- acide 8-chloro-7-fluoro-9-[4-(3-fluoro-4-méthylphényl)pipérazin-1-yl]-4-méthyl-1-oxo-1,4-dihydrobenzo[f][1,7]naphtyridine-2-carboxylique 2,52 mg
- cetomacrogol 30,0 mg
- alcool benzylique 3,0 mg
- eau ppi 100 mg

Par ailleurs, les produits de formule générale (I) peuvent également être utilisés comme agents de conservation ou de désinfection des matières organiques ou minérales. Notamment dans l'industrie des colorants, de matières grasses, du papier, du bois, des polymères ou encore dans l'industrie textile, l'industrie alimentaire ou le traitement des eaux. Il est également entendu que les compositions renfermant un produit de formule générale (I) à l'état pur ou sous forme d'association avec des diluants ou adjuvants compatibles, entrent aussi dans le cadre de la présente invention.

## Revendications

1. Dérivé de la benzo[f]naphtyridine de formule générale : 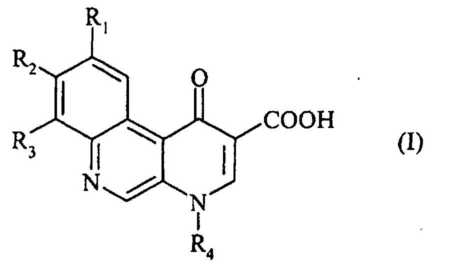 dans laquelle,
- R₁, R₂ et R₃, identiques ou différents, représentent un atome d'hydrogène, un atome d'halogène ou bien un groupe de formule générale (II) : 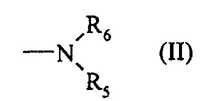 dans laquelle R₅ et R₆ forment ensemble avec l'atome d'azote auquel ils sont rattachés un hétérocycle à 5, 6 ou 7 chaînons dont 2 atomes de carbone peuvent éventuellement être liés entre eux par un pont contenant 1 ou 2 atomes de carbone, et contenant le cas échéant un autre hétéroatome choisi parmi l'azote, l'oxygène ou le soufre, ledit cycle pouvant en outre être substitué par un ou plusieurs radicaux phényle, phényle substitué (par un atome d'halogène, un radical alkyle, halogénoalkyle, alkyloxy, ou benzyloxy), benzyle, alkyle, hydroxy, aminoalkyle, alkylaminoalkyle, dialkylaminoalkyle, ou benzylaminoalkyle,
étant entendu que l'un des substituants R₁, R₂ ou R₃ est nécessairement un groupe de formule générale (II) -NR₅R₆, et l'un au moins des deux autres représente un atome d'halogène, et
- R₄ représente un radical alkyle, fluoroalkyle, carboxyalkyle, cycloalkyle contenant 3 à 6 atomes de carbone, fluorophényle, difluorophényle, alkyloxy ou alkylamino, les radicaux alkyle cités ci-dessus étant droits ou ramifiés et contenant 1 à 4 atomes de carbone,
sous ses formes stéréoisomères ou leurs mélanges, ainsi que ses sels métalliques, ses sels d'addition avec les bases azotées et ses sels d'addition avec les acides.

2. Dérivé de la benzo[f]naphtyridine selon la revendication 1, de formule générale: 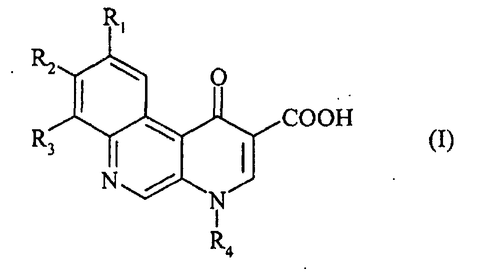 dans laquelle :
- R₁, R₂ et R₃, identiques ou différents, représentent un atome d'hydrogène, un atome d'halogène ou bien un groupe de formule générale (II) : 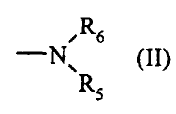
dans laquelle R₅ et R₆ forment ensemble avec l'atome d'azote auquel ils sont rattachés un hétérocycle à 6 ou 7 chaînons dont 2 atomes de carbone peuvent éventuellement être liés entre eux par un pont contenant 1 ou 2 atomes de carbone, et contenant le cas échéant un autre hétéroatome d'azote, ledit cycle pouvant en outre être substitué par un ou plusieurs radicaux phényle, phényle substitué (par un atome d'halogène, un radical alkyle, halogénoalkyle, alkyloxy, ou benzyloxy), ou alkyle, étant entendu que l'un des substituants R₁, R₂ ou R₃ est nécessairement un groupe de formule générale (II), et l'un au moins des deux autres représente un atome d'halogène, et
- R₄ représente un radical alkyle ou fluoroalkyle,
les radicaux alkyle cités ci-dessus étant droits ou ramifiés et contenant 1 à 4 atomes de carbone,
sous ses formes stéréoisomères ou leurs mélanges, ainsi que ses sels métalliques, ses sels d'addition avec les bases azotées et ses sels d'addition avec les acides.

3. Procédé de préparation d'un dérivé de la benzo[f]naphtyridine selon la revendication 1, **caractérisé en ce que** l'on fait agir une amine de formule générale : 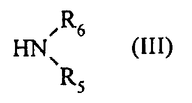 dans laquelle R₅ et R₆ sont définis comme dans la revendication 1, sur un dérivé de benzo[f]naphtyridine de formule générale : 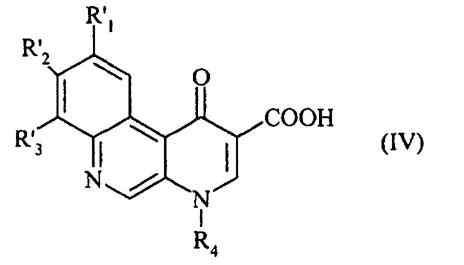 dans laquelle R₄ est défini comme dans la revendication 1 ou représente un radical alkylamino protégé, et R'₁, R'₂ et R'₃ représentent indépendamment les uns des autres un atome d'hydrogène ou un atome d'halogène, étant entendu que deux au moins des substituants R'₁, R'₂ et R'₃ sont des atomes d'halogène,
les atomes d'halogène cités ci-dessus étant choisis parmi le fluor, le chlore, le brome ou l'iode,
puis sépare les produits aminés ainsi obtenus pour sélectionner la structure souhaitée, et éventuellement transforme le produit obtenu en un sel.

4. Procédé de préparation d'un dérivé de la benzo[f]naphtyridine selon la revendication 1 ou 2, **caractérisé en ce que** l'on transforme l'ester de formule générale : 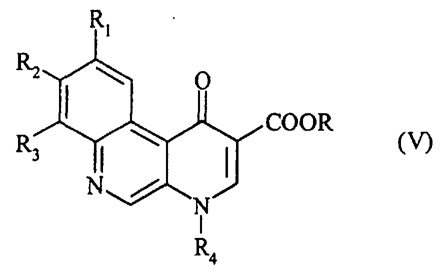 dans laquelle R₁, R₂ et R₃ sont définis comme dans la revendication 1 ou 2, R₄ est défini comme dans la revendication 1 ou 2 ou bien représente un radical alkylamino protégé et R représente un radical alkyle contenant 1 à 4 atomes de carbone en chaîne droite ou ramifiée, par toute méthode connue pour obtenir un acide à partir d'un ester, sans toucher au reste de la molécule, puis, le cas échéant, élimine le groupement protecteur du radical alkylamino, et/ou éventuellement prépare le sel du dérivé de benzo[f]naphtyridine obtenu.

5. Procédé de préparation d'un dérivé de benzo[f|naphtyridine selon la revendication 4 **caractérisé en ce que** le dit ester de formule générale : 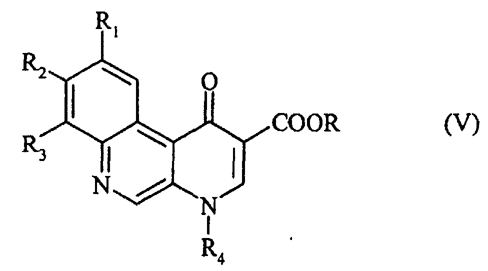 dans laquelle R₁, R₂ et R₃ sont définis comme dans la revendication 1 ou 2, R₄ est défini comme dans la revendication 1 ou 2 ou bien représente un radical alkylamino protégé et R est défini comme dans la revendication 4, est obtenu par action d'une aminé de formule générale : 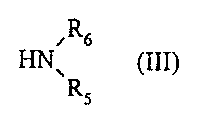 dans laquelle R₅ et R₆ sont définis comme dans la revendication 1 ou 2, sur un dérivé de benzo[f]naphtyridine de formule générale : 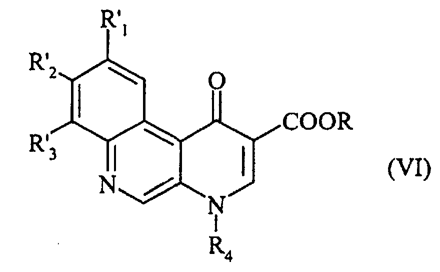 dans laquelle R'₁, R'₂ et R'₃ sont définis comme dans la revendication 3, et R et R₄ sont définis comme dans la revendication 4.

6. Procédé de préparation d'un dérivé de la benzo[f]naphtyridine selon la revendication 5, **caractérisé en ce que** le dérivé de formule générale : 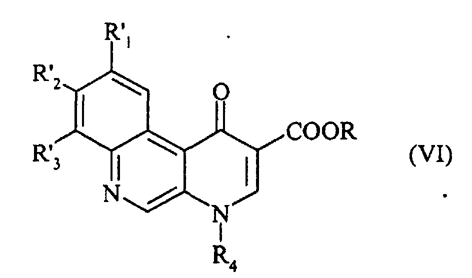 dans laquelle R'₁, R'₂ et R'₃ sont définis comme dans la revendication 3, et R et R₄ sont définis comme dans la revendication 4, est obtenu de la façon suivante :
a) on fait agir un dérivé de malonate de formule générale : 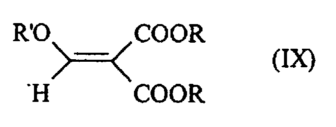 dans laquelle R est tel que défini dans la revendication 4 et R', identique ou différent de R, représente un radical alkyle contenant 1 à 4 atomes de carbone en chaîne droite ou ramifiée, sur une aminoquinoléine de formule générale : 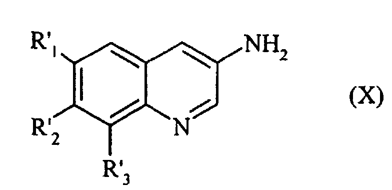 dans laquelle R'₁, R'₂ et R'₃ sont tels que défini dans la revendication 3,
b) on cyclise le dérivé résultant de l'étape a) de formule générale : 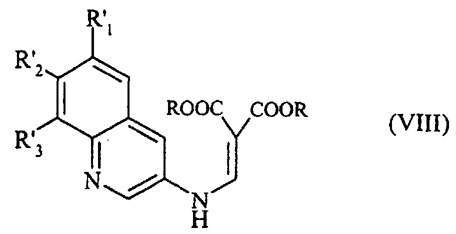 dans laquelle R'₁, R'₂ et R'₃ sont tels que défini dans la revendication 3, et R est tel que défini dans la revendication 4, par cyclisation thermique de type Gould-Jacob,
c) puis on additionne un dérivé halogéné de formule R₄-Hal, pour lequel R₄ est défini comme dans la revendication 1 ou 2 et Hal représente un atome d'halogène choisi parmi le chlore, le brome ou l'iode, sur le dérivé obtenu à l'étape b) de formule générale : 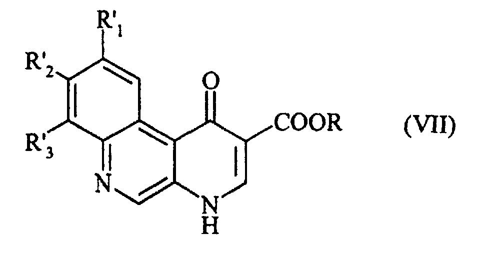
dans laquelle R'₁, R'₂ et R'₃ sont tels que défini dans la revendication 3, et R est tel que défini dans la revendication 4.

7. Un dérivé de benzo[f]naphtyridine de formule générale : 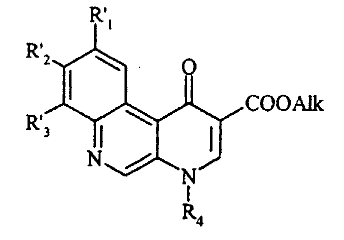 dans laquelle R'₁, R'₂ et R'₃ sont tels que défini dans la revendication 3, R₄ est tel que défini dans la revendication 4, et Alk représente un atome d'hydrogène ou bien un radical alkyle droit ou ramifié et contenant 1 à 4 atomes de carbone.

8. Un dérivé d'aminoquinoléine de formule générale : 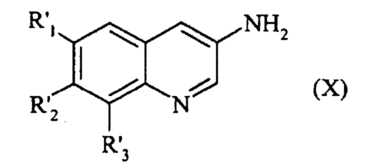 dans laquelle R'₁, R'₂ et R'₃ sont tels que définis dans la revendication 3.

9. Composition **caractérisée en ce qu'**elle contient au moins un dérivé selon la revendication 1 ou 2, à l'état pur ou en association avec un ou plusieurs diluants ou adjuvants compatibles.

## Patentansprüche

1. Benzo[f]-naphthyridin-Derivate der allgemeinen Formel (I) 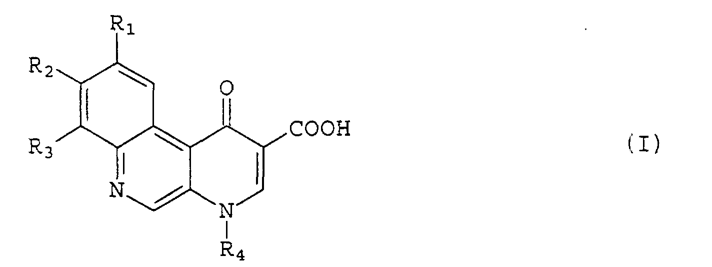 in der
- R₁, R₂ und R₃, gleich oder verschieden, ein Wasserstoffatom, ein Halogenatom oder eine Gruppe der allgemeinen Formel (II) 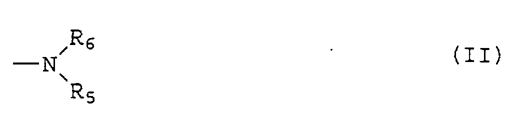 darstellen, in der R₅ und R₆ zusammen mit dem Stickstoffatom, an das sie gebunden sind, einen Heterocyclus mit 5, 6 oder 7 Ringgliedern bilden, von denen 2 Kohlenstoffatome gegebenenfalls untereinander durch eine Brücke verbunden sein können, die 1 oder 2 Kohlenstoffatome enthält, wobei der Heterocyclus gegebenenfalls ein weiteres Heteroatom enthält, ausgewählt unter Stickstoff, Sauerstoff und Schwefel, und der genannte Ring außerdem substituiert sein kann durch einen oder mehrere Reste Phenyl, Phenyl substituiert (durch ein Halogenatom, einen Rest Alkyl, Halogenalkyl, Alkyloxy oder Benzyloxy), Benzyl, Alkyl, Hydroxy, Aminoalkyl, Alkylaminoalkyl, Dialkylaminoalkyl oder Benzylaminoalkyl, mit der Maßgabe, daß einer der Substituenten R₁, R₂ oder R₃ zwangsläufig eine Gruppe der allgemeinen Formel (II) -NR₅R₆ ist und mindestens einer der zwei anderen ein Halogenatom darstellt, und
- R₄ einen Rest Alkyl, Fluoralkyl, Carboxyalkyl, Cycloalkyl mit 3 bis 6 Kohlenstoffatomen, Fluorphenyl, Difluorphenyl, Alkyloxy oder Alkylamino bedeutet, wobei die oben genannten Reste Alkyl gerade oder verzweigt sind und 1 bis 4 Kohlenstoffatome enthalten,
in allen ihren stereoisomeren Formen oder ihren Mischungen sowie ihren Metallsalzen, ihren Additionssalzen mit Stickstoffbasen und ihren Additionssalzen mit Säuren.

2. Benzo[f]-naphthyridin-Derivate nach Anspruch 1 der allgemeinen Formel (I) 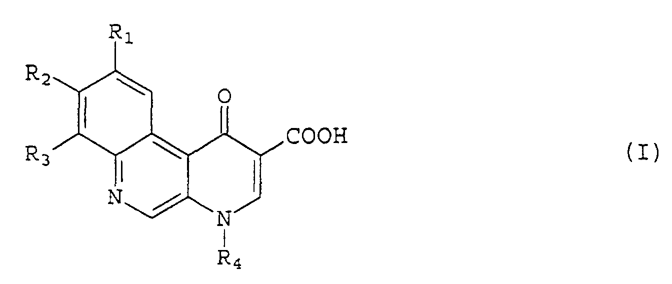 in der
- R₁, R₂ und R₃, gleich oder verschieden, ein Wasserstoffatom, ein Halogenatom oder eine Gruppe der allgemeinen Formel (II) 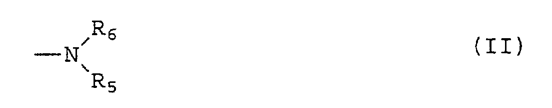 darstellen, in der R₅ und R₆ zusammen mit dem Stickstoffatom, an das sie gebunden sind, einen Heterocyclus mit 6 oder 7 Ringgliedern bilden, von denen 2 Kohlenstoffatome gegebenenfalls untereinander durch eine Brücke verbunden sein können, die 1 oder 2 Kohlenstoffatome enthält, wobei der Heterocyclus gegebenenfalls ein weiteres Heteroatom von Stickstoff enthält, und der genannte Ring außerdem substituiert sein kann durch einen oder mehrere Reste Phenyl, Phenyl substituiert (durch ein Halogenatom, einen Rest Alkyl, Halogenalkyl, Alkyloxy oder Benzyloxy) oder Alkyl, mit der Maßgabe, daß einer der Substituenten R₁, R₂ oder R₃ zwangsläufig eine Gruppe der allgemeinen Formel (II) ist und mindestens einer der zwei anderen ein Halogenatom darstellt, und
- R₄ einen Rest Alkyl oder Fluoralkyl bedeutet, wobei die oben genannten Reste Alkyl gerade oder verzweigt sind und 1 bis 4 Kohlenstoffatome enthalten, in allen ihren stereoisomeren Formen oder ihren Mischungen sowie ihren Metallsalzen, ihren Additionssalzen mit Stickstoffbasen und ihren Additionssalzen mit Säuren.

3. Verfahren zur Herstellung eines Benzo[f]-naphthyridin-Derivates nach Anspruch 1, **dadurch gekennzeichnet, daß** man ein Amin der allgemeinen Formel (III) 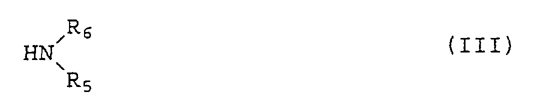 in der R₅ und R₆ wie in Anspruch 1 definiert sind, mit einem Benzo[f]-naphthyridin-Derivat der allgemeinen Formel (IV) 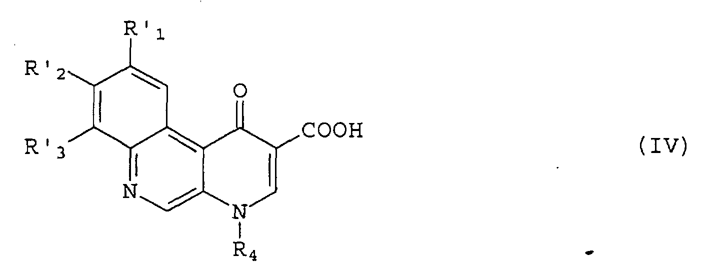 zur Reaktion bringt, in der R₄ wie in Anspruch 1 definiert ist oder einen geschützten Rest Alkylamino darstellt, und R'₁, R'₂ und R'₃ unabhängig voneinander ein Wasserstoffatom oder ein Halogenatom bedeuten, mit der Maßgabe, daß mindestens zwei der Substituenten R'₁, R'₂ und R'₃ Halogenatome sind, wobei die oben genannten Halogenatome unter Fluor, Chlor, Brom oder Iod ausgewählt werden,
und man anschließend die auf diese Weise erhaltenen aminierten Produkte trennt, um die gewünschte Struktur zu selektieren, und das erhaltene Produkt gegebenenfalls in ein Salz überführt.

4. Verfahren zur Herstellung eines Benzo[f]-naphthyridin-Derivates nach Anspruch 1 oder 2, **dadurch gekennzeichnet, daß** man den Ester der allgemeinen Formel (V) 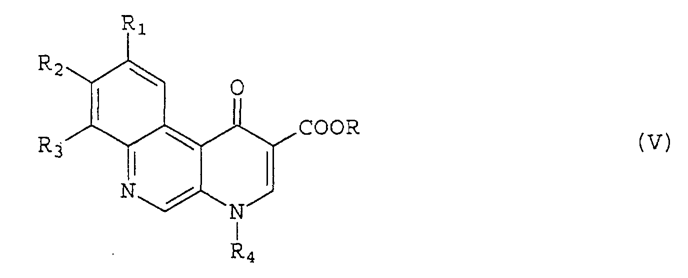 in der R₁, R₂ und R₃ wie in Anspruch 1 oder 2 definiert sind, R₄ wie in Anspruch 1 oder 2 definiert ist oder einen geschützten Rest Alkylamino darstellt und R einen Rest Alkyl mit 1 bis 4 Kohlenstoffatomen in gerader oder verzweigter Kette bedeutet, nach jeder bekannten Methode umwandelt, um eine Säure ausgehend von einem Ester zu erhalten, ohne den Rest des Moleküls zu beeinträchtigen, und man anschließend gegebenenfalls die Schutzgruppe von dem Rest Alkylamino entfernt und/oder gegebenenfalls das Salz des erhaltenen Benzo [f]-naphthyridin-Derivates herstellt.

5. Verfahren zur Herstellung eines Benzo[f]-naphthyridin-I)erivates nach Anspruch 4, **dadurch gekennzeichnet, daß** der genannte Ester der allgemeinen Formel (V) 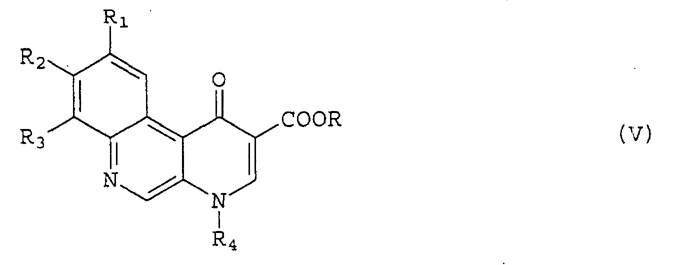 in der R₁, R₂ und R₃ wie in Anspruch 1 oder 2 definiert sind, R₄ wie in Anspruch 1 oder 2 definiert ist oder einen geschützten Rest Alkylamino darstellt und R wie in Anspruch 4 definiert ist, durch Einwirkung eines Amins der allgemeinen Formel (III) 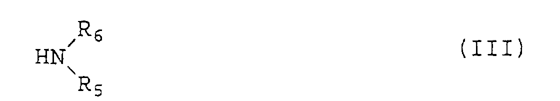 in der R₅ und R₆ wie in Anspruch 1 oder 2 definiert sind, auf ein Benzo[f]-naphthyridin-Derivat der allgemeinen Formel (VI) 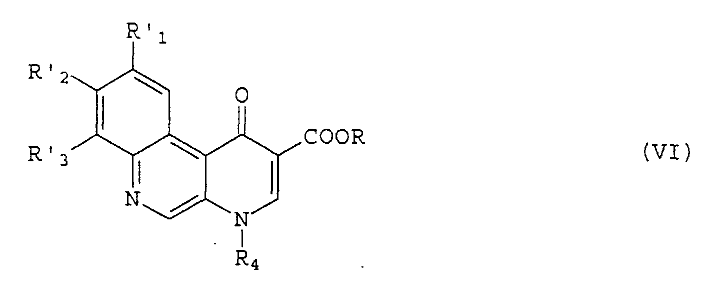 erhalten wird, in der R'₁, R'₂ und R'₃ wie in Anspruch 3 definiert sind und R und R₄ wie in Anspruch 4 definiert sind.

6. Verfahren zur Herstellung eines Benzo[f]-naphthyridin-Derivates nach Anspruch 5, **dadurch gekennzeichnet, daß** das Derivat der allgemeinen Formel (VI) 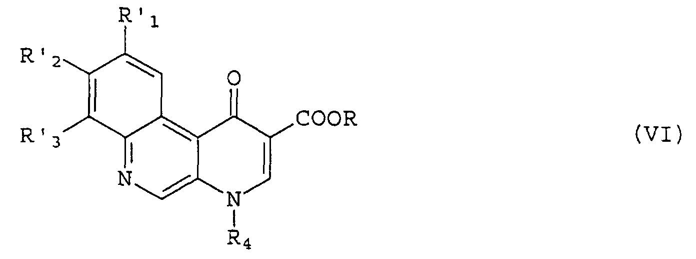 in der R'₁, R'₂ und R'₃ wie in Anspruch 3 definiert sind und R und R₄ wie in Anspruch 4 definiert sind, in der folgenden Art und Weise erhalten wird:
a) man bringt ein Malonat-Derivat der allgemeinen Formel (IX) 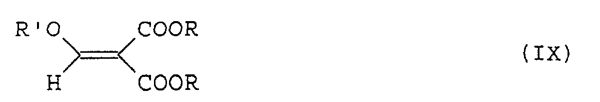 in der R wie Anspruch 4 definiert ist und R', gleich mit R oder verschieden von R, einen Rest Alkyl mit 1 bis 4 Kohlenstoffatomen in gerader oder verzweigter Kette darstellt, mit einem Aminochinolin der allgemeinen Formel (X) 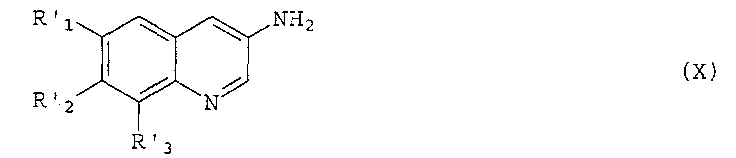 zur Reaktion, in der R'₁, R'₂ und R'₃ wie in Anspruch 3 definiert sind,
b) man cyclisiert das in Stufe a) erhaltene Derivat der allgemeinen Formel (VIII) 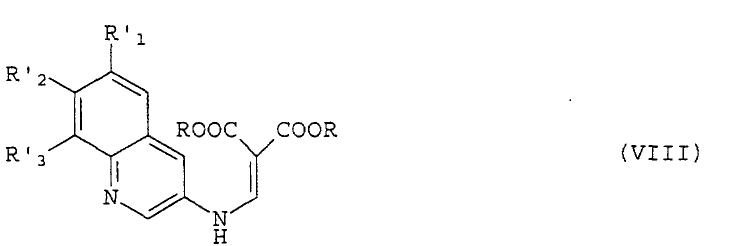 in der R'₁, R'₂ und R'₃ wie in Anspruch 3 definiert sind und R wie in Anspruch 4 definiert ist, durch thermische Cyclisierung vom Typ Gould-Jacob,
c) man addiert anschließend ein Halogenderivat der Formel R₄-Hal, worin R₄ wie in Anspruch 1 oder 2 definiert ist und Hal ein Halogenatom darstellt, ausgewählt unter Chlor, Brom und Iod, mit dem in Stufe b) erhaltenen Derivat der allgemeinen Formel (VII) 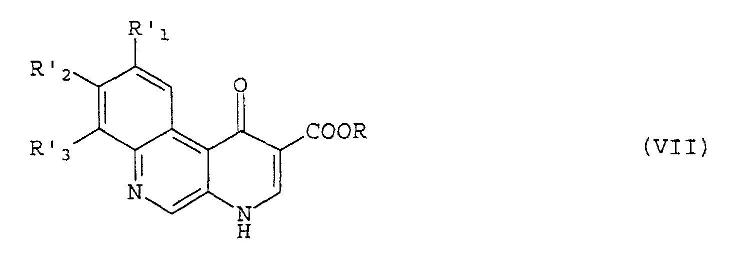 in der R'₁, R'₂ und R'₃ wie in Anspruch 3 definiert sind und R wie in Anspruch 4 definiert ist.

7. Benzo[f]-naphthyridin-Derivat der allgemeinen Formel 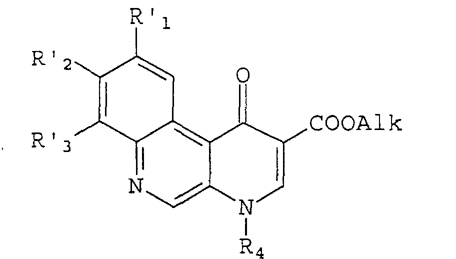 in der R'₁, R'₂ und R'₃ wie in Anspruch 3 definiert sind, R₄ wie in Anspruch 4 definiert ist und Alk ein Wasserstoffatom oder einen geraden oder verzweigten Rest Alkyl mit 1 bis 4 Kohlenstoffatomen darstellt.

8. Aminochinolin-Derivat der allgemeinen Formel (X) 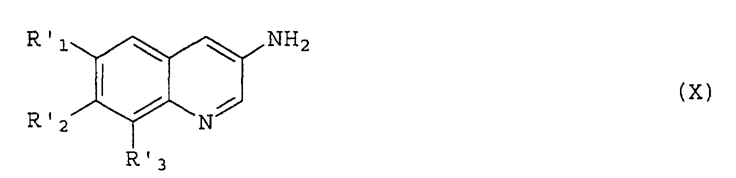 in der R'₁, R'₂ und R'₃ wie in Anspruch 3 definiert sind.

9. Zusammensetzung, **dadurch gekennzeichnet, daß** sie mindestens ein Derivat nach Anspruch 1 oder 2 in reinem Zustand oder in Assoziation mit einem oder mehreren kompatiblen Verdünnungsmitteln oder Zusatzstoffen enthält.

## Claims

1. Benzo[f]naphthyridine derivative of general formula: 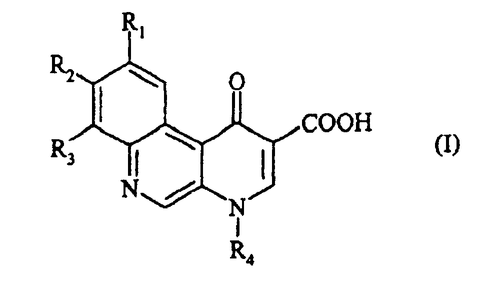 in which,
- R₁, R₂ and R₃, which are identical or different, represent a hydrogen atom, a halogen atom or a group of general formula (II) : 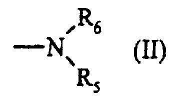 in which R₅ and R₆ form together with the nitrogen atom to which they are attached a 5-, 6- or 7-membered heterocycle in which 2 carbon atoms may optionally be linked to each other by a bridge containing 1 or 2 carbon atoms, and containing, where appropriate, another heteroatom chosen from nitrogen, oxygen or sulphur, it being possible, in addition, for the said ring to be substituted with one or more phenyl, substituted phenyl (substituted with a halogen atom, an alkyl, haloalkyl, alkyloxy or benzyloxy radical), benzyl, alkyl, hydroxyl, aminoalkyl, alkylaminoalkyl, dialkylaminoalkyl or benzylaminoalkyl radicals, it being understood that one of the substituents R₁, R₂ or R₃ is necessarily a group of general formula (II) -NR₅R₆, and at least one of the other two represents a halogen atom, and
- R₄ represents an alkyl, fluoroalkyl, carboxyalkyl, cycloalkyl containing 3 to 6 carbon atoms, fluorophenyl, difluorophenyl, alkyloxy or alkylamino radical,
the abovementioned alkyl radicals being straight or branched and containing 1 to 4 carbon atoms,
in its stereoisomeric forms or mixtures thereof, as well as its metal salts, its addition salts with nitrogenous bases and its addition salts with acids.

2. Benzo[f]naphthyridine derivative according to Claim 1, of general formula: 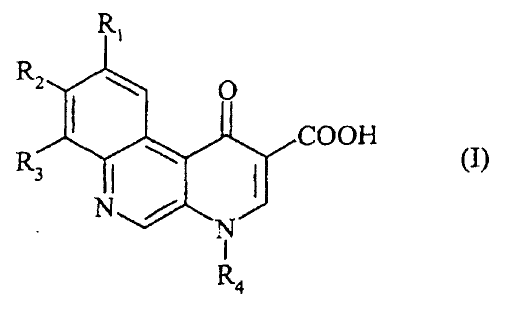 in which:
- R₁, R₂ and R₃, which are identical or different, represent a hydrogen atom, a halogen atom or a group of general formula (II): 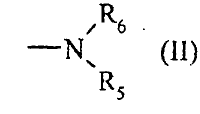 in which R₅ and R₆ form together with the nitrogen atom to which they attached a 6- or 7-membered heterocycle in which 2 carbon atoms may be optionally linked to each other by a bridge containing 1 or 2 carbon atoms, and containing, where appropriate, another nitrogen heteroatom, it being possible, in addition, for the said ring to be substituted with one or more phenyl, substituted phenyl (substituted with a halogen atom, an alkyl, haloalkyl, alkyloxy or benzyloxy radical), or alkyl radicals,
it being understood that one of the substituents R₁, R₂ or R₃ is necessarily a group of general formula (II), and at least one of the other two represents a halogen atom, and
- R₄ represents an alkyl or fluoroalkyl radical, the abovementioned alkyl radicals being straight or branched and containing 1 to 4 carbon atoms,
in its stereoisomeric forms or. mixtures thereof, as well as its metal salts, its addition salts with nitrogenous bases and its addition salts with acids.

3. Process for the preparation of a benzo[f]naphthyridine derivative according to Claim 1, **characterized in that** an amine of general formula: 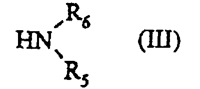 in which R₅ and R₆ are defined as in Claim 1, is reacted with a benzo[f]naphthyridine derivative of general formula: 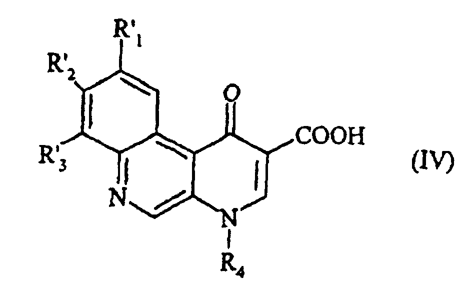 in which R₄ is defined as in Claim 1 or represents a protected alkylamino radical, and R'₁, R'₂ and R'₃ represent, independently of each other, a hydrogen atom or a halogen atom, it being understood that at least two of the substituents R'₁, R'₂ and R'₃ are halogen atoms,
the abovementioned halogen atoms being chosen from fluorine, chlorine, bromine or iodine, and then the amine-containing products thus obtained are separated in order to select the desired structure, and the product obtained is optionally converted to a salt.

4. Process for the preparation of a benzo[f]naphthyridine derivative according to Claim 1 or 2, **characterized in that** the ester of general formula: 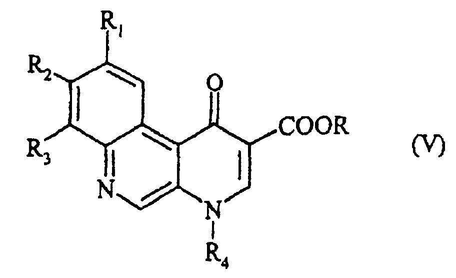 in which R₁, R₂ and R₃ are defined as in Claim 1 or 2, R₄ is defined as in Claim 1 or 2 or represents a protected alkylamino radical and R represents an alkyl radical containing 1 to 4 straight- or branched-chain carbon atoms, is converted by any known method for obtaining an acid from an ester, without affecting the remainder of the molecule, and then, where appropriate, the alkylamino radical-protecting group is removed, and/or the salt of the benzo[f]naphthyridine derivative obtained is optionally prepared.

5. Process for the preparation of a benzo[f]naphthyridine derivative according to Claim 4, **characterized in that** the said ester of general formula: 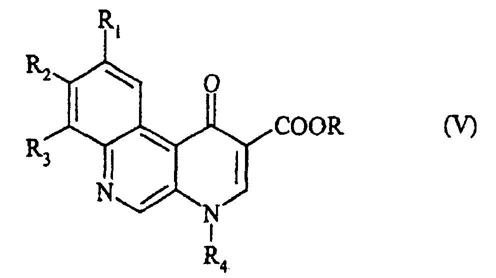 in which R₁, R₂ and R₃ are defined as in Claim 1 or 2, R₄ is defined as in Claim 1 or 2 or represents a protected alkylamino radical and R is defined as in Claim 4, is obtained by reacting an amine of general formula: 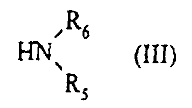 in which R₅ and R₆ are defined as in Claim 1 or 2, with a benzo[f]naphthyridine derivative of general formula: 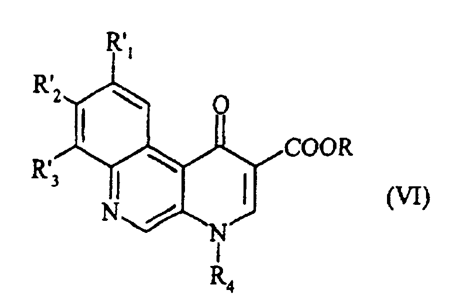 in which R'₁, R'₂ and R'₃ are defined as in Claim 3, and R and R₄ are defined as in Claim 4.

6. Process for the preparation of a benzo[f]naphthyridine derivative according to Claim 5,
**characterized in that** the derivative of general formula: 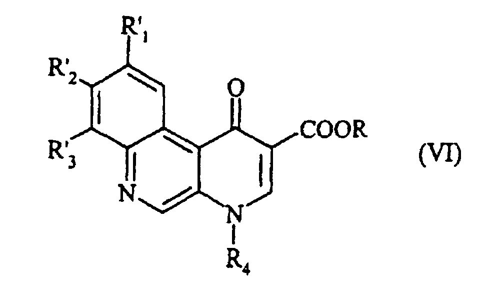 in which R'₁, R'₂ and R'₃ are defined as in Claim 3, and R and R₄ are defined as in Claim 4, is obtained in the following manner:
a) a malonate derivative of general formula: 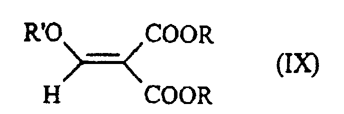 in which R is as defined in Claim 4 and R', which is identical to or different from R, represents an alkyl radical containing 1 to 4 straight- or branched-chain carbon atoms, is reacted with an aminoquinoline of general formula: 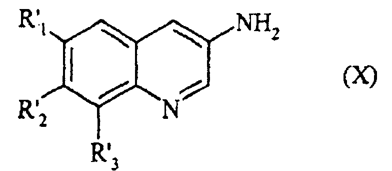 in which R'₁, R'₂ and R'₃ are as defined in Claim 3,
b) the derivative, resulting from step a), of general formula: 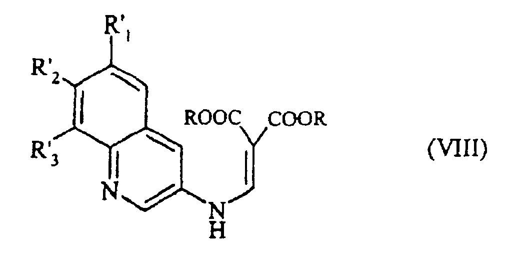 in which R'₁, R'₂ and R'₃ are as defined in Claim 3, and R is as defined in Claim 4, is cyclized by thermal cyclization of the Gould-Jacob type,
c) and then a halogenated derivative of formula R₄-Hal, for which R₄ is defined as in Claim 1 or 2 and Hal represents a halogen atom chosen from chlorine, bromine or iodine, is added to the derivative obtained in step b), of general formula: 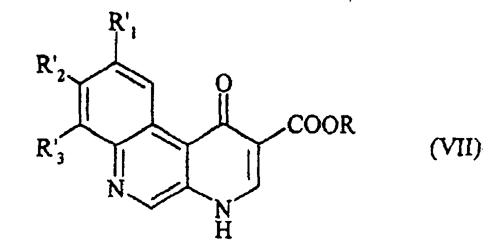 in which R'₁, R'₂ and R'₃ are as defined in Claim 3, and R is as defined in Claim 4.

7. Benzo[f]naphthyridine derivative of general formula: 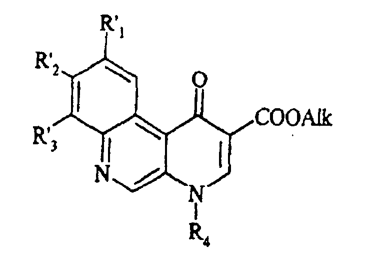 in which R'₁, R'₂ and R'₃ are as defined in Claim 3, R₄ is as defined in Claim 4, and Alk represents a hydrogen atom or a straight or branched alkyl radical containing 1 to 4 carbon atoms.

8. Aminoquinoline derivative of general formula: 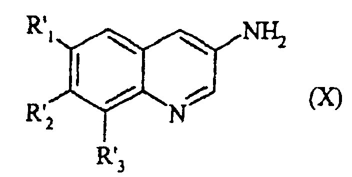 in which R'₁, R'₂ and R'₃ are as defined in Claim 3.

9. Composition **characterized in that** it contains at least one derivative according to Claim 1 or 2, in the pure state or in combination with one or more compatible diluents or adjuvants.
